(19) Europäisches Patentamt
European Patent Office
Office européen des brevets

(11) EP 0 693 900 B1

(12) EUROPEAN PATENT SPECIFICATION

(45) Date of publication and mention of the grant of the patent:
**17.03.2004 Bulletin 2004/12**

(21) Application number: **93910585.4**

(22) Date of filing: **12.04.1993**

(51) Int Cl.$^7$: **A61B 5/00**, G01N 21/31

(86) International application number:
**PCT/US1993/003427**

(87) International publication number:
**WO 1994/023643 (27.10.1994 Gazette 1994/24)**

(54) **SYSTEM AND METHOD FOR NONINVASIVE HEMATOCRIT MONITORING**

GERÄT UND VERFAHREN ZUR NICHTINVASIVEN ÜBERWACHUNG DES HÄMATOCRIT-WERTES

SYSTEME ET PROCEDE DE SURVEILLANCE NON INVASIVE DE L'HEMATOCRITE

(84) Designated Contracting States:
**AT BE CH DE ES FR GB IT LI NL SE**

(43) Date of publication of application:
**31.01.1996 Bulletin 1996/05**

(73) Proprietor: **Hema Metrics, Inc.**
**Riverdale, UT 84405 (US)**

(72) Inventors:
- **STEUER, Robert R.**
  **Pleasant View, UT 84414 (US)**
- **HARRIS, David H.**
  **Boise, ID 83713 (US)**

(74) Representative: **Gray, John James et al**
**Fitzpatricks,**
**4 West Regent Street**
**Glasgow G2 1RS (GB)**

(56) References cited:

**WO-A-86/06946**
**WO-A-93/11701**
**WO-A-93/13706**
**US-A- 3 638 604**
**US-A- 4 086 915**
**US-A- 4 805 623**
**US-A- 4 925 299**
**US-A- 5 048 524**
**US-A- 5 054 487**
**US-A- 5 066 859**
**US-A- 5 101 825**

- **SCHMITT J.M. ET AL: 'Measurement of blood hematocrit by dual-wavelength near-IR photoplethysmography' PROCEEDINGS OF THE SPIE vol. 1641, 22 January 1992, VA, US, pages 150 - 161, XP000573159**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 693 900 B1

## Description

This invention relates to systems and methods for noninvasively measuring hematocrit. More particularly, the present invention relates to noninvasive spectrophotometric systems and methods for quantitatively and continuously monitoring the hematocrit of a subject.

Modern medical practice utilizes a number of procedures and indicators to assess a patient's condition. One of these indicators is the patient's hematocrit. Hematocrit (often abbreviated as Hct) is the volume, expressed as a percentage, of the patient's blood which is occupied by red corpuscles (commonly referred to as red blood cells).

Human blood consists principally of liquid plasma (which is comprised of over 90% water with more than 100 other constituents such as proteins, lipids, salts, etc.) and three different corpuscles. The three corpuscles found in blood are red corpuscles, white corpuscles, and platelets.

The chief function of red corpuscles is to carry oxygen from the lungs to the body tissues and carbon dioxide from the tissues to the lungs. This critical life supporting function is made possible by hemoglobin which is the principal active constituent of red corpuscles. In the lungs, hemoglobin rapidly absorbs oxygen to form oxyhemoglobin which gives it a bright scarlet color. As the red corpuscles travel to the tissues, the oxyhemoglobin releases oxygen, i.e., is reduced, and the hemoglobin turns a dark red color.

The oxygen transportation functions of the body rely essentially entirely on the presence of hemoglobin in the red corpuscles. Red corpuscles greatly outnumber other corpuscles being about 700 times greater than the number of white corpuscles in a healthy human subject.

Medical professionals routinely desire to know the hematocrit of a patient. In order to determine hematocrit using any of the techniques available to date, it is necessary to draw a sample of blood by puncturing a vein or invading a capillary. Then, using a widely accepted technique, the sample of blood is subjected to a high speed centrifuge treatment for several minutes (e.g., 7 or more minutes). The centrifuging process, if properly carried out, separates the corpuscles into a packed mass. The volume occupied by the packed corpuscles, expressed as a percentage of the total volume of the plasma/corpuscle combination, is taken as the hematocrit.

It will be appreciated that the centrifuge process provides a hematocrit value which includes all corpuscles, not just red corpuscles. Nevertheless, the vastly greater numbers of red corpuscles in a healthy subject allows the hematocrit value obtained by the centrifuge process to be clinically usable in such healthy subjects. Nevertheless, in subjects with low hematocrit or dramatically high white corpuscle content, it may be desirable to diminish the effect of the non-red corpuscles when obtaining an hematocrit value.

There have been various techniques and devices introduced which have automated and increased the precision of obtaining a hematocrit value. Nevertheless, all the previously available techniques have one or more drawbacks.

Specifically, the previously available techniques all require that a sample of blood be withdrawn from the patient for in vitro analysis. Any invasion of the subject to obtain blood is accompanied by the problems of inconvenience, stress, and discomfort imposed upon the subject and also the risks which are always present when the body is invaded. Drawing blood, also creates certain contamination risks to the paramedical professional. Moreover, even in a setting where obtaining a blood sample does not impose any additional problems, e.g., during surgery, the previously available techniques, require a delay between the time that the sample is drawn and the hematocrit value is obtained. Still further, none of the previously available techniques allow continuous monitoring of a subject's hematocrit, as might be desirable during some surgical procedures or intensive care treatment, but require the periodic withdrawal and processing of blood samples.

In view of the drawbacks inherent in the available art dealing with invasive hematocrit determinations, it would be an advance in the art to noninvasively and quantitatively determine a subject's hematocrit value. It would also be an advance in the art to provide a system and method for noninvasive hematocrit monitoring which can be applied to a plurality of body parts and which utilizes electromagnetic emissions as an hematocrit information carrier. It would be another advance in the art to provide a system and method which can provide both immediate and continuous hematocrit information for a subject. It would be yet another advance to provide repeatable and reliable systems for noninvasive monitoring of a subject's hematocrit. It would be still another advance in the art to noninvasively and accurately determine a subjects blood oxygen saturation while accounting for the patient's low or varying hematocrit and/or under conditions of low perfusion.

Various techniques for (noninvasive optical oximetry have been disclosed previously, for example in US3,638,640 and US4,086,915 and others mentioned in the detailed description, below.

The present invention, defined in the appended claims, is directed to systems and methods for determining a hematocrit value, transcutaneously and noninvasively. This is achieved by passing at least two wavelengths of light onto or through body tissues such as the finger, earlobe, or scalp, etc. and then compensating for the effects body tissue and fluid effects. As used herein, the term biologic constituent includes proteins, red cells, metabolites, drugs, cytochromes, hormones, etc.

In one embodiment within the scope of the present invention, the wavelengths of light are selected to be near or at the isobestic points of reduced hemoglobin and oxyhemoglobin to eliminate the effects of variable blood oxygenation. At an isobestic wavelength, the extinction coefficient, $\varepsilon$, is the same for both reduced and oxygenated hemoglobin. Thus, at isobestic wavelengths, the amount of light absorption is independent of the amount of oxygenated or reduced hemoglobin in the red cells.

Means are provided for delivering and detecting those wavelengths of light and for analyzing the light intensities. The sensing and radiation emitting elements are preferably spatially arranged to allow ease of use and to be accessible to a patient's exterior body parts. The configuration of the sensing and emitting elements is important to give optimum repeatability of the signals and data derived therefrom.

Memory and calculation means are included which are capable of storing, manipulating, and displaying the detected signals in a variety of ways. For instance, the continuous pulse wave contour, the pulse rate value, the hematocrit value and the continuous analog hematocrit curve in real time, the hematocrit-independent oxygen saturation value, and the oxygen content value of the blood, all as digital values or as continuous analog curves in real time are capable of being displayed.

An important advantage of monitoring and analyzing each individual pulsatile signal is that averaging algorithms may be performed for identifying and rejecting erroneous data. In addition, such techniques also improve repeatability.

Another significant advantage of the present invention is the capability of monitoring multiple wavelengths (including nonisobestic wavelengths) for the simultaneous real time computation and display of the hematocrit-independent oxygen saturation value. Techniques in prior art oximetry have all suffered inaccuracies due to hematocrit sensitivities.

Rather than apply AC-DC cancellation techniques only, it is also within the scope of the present invention to detect and analyze multiple wavelengths using a logarithmic DC analysis technique. In this embodiment, a pulse wave is not required. Hence, this embodiment may be utilized in states of low blood pressure or low blood flow.

## BRIEF DESCRIPTION OF THE DRAWINGS

Figure 1 is a perspective view of a system according to a first presently preferred embodiment of the present invention.

Figure 1A is an enlarged cross sectional view of the body part (finger) and system components represented in Figure 1 used in a transmission mode.

Figure 1B is an enlarged cross sectional view of the body part (finger) and associated system components represented in Figure 1 used in a reflective mode.

Figure 2 is a chart showing the optical absorption coefficients of oxyhemoglobin ($HbO_2$), reduced hemoglobin (Hb), and water ($H_2O$) versus wavelength.

Figure 3 is a chart showing the relationship between the extinction coefficient of light at three different wavelengths versus hematocrit for whole blood.

Figure 4 is a chart showing the relationship between the ratio of the extinction coefficients of two rays having differing wavelengths versus hematocrit.

Figures 5A-5D provide a flow chart showing the steps carried out during one presently preferred method of the present invention using the pulsatile component of the subject's blood flow to provide accurate hematocrit and blood oxygen saturation values.

Figure 6 is a perspective view of a second presently preferred system of the present invention which is applied to the ear and includes structures to squeeze out the blood to blanch the ear tissues.

Figure 6A is an enlarged cross sectional view of the ear and system components represented in Figure 6.

Figure 7 provides a detailed schematic diagram of the low level sensor circuitry included in the presently preferred system of the present invention.

Figures 8A-8C provide a detailed schematic diagram digital section circuitry included in the presently preferred system of the present invention.

Figures 9A-9D provide a detailed schematic diagram of the analog section circuitry included in the presently preferred system of the present invention.

Figures 10A-10C provide a detailed schematic diagram of the power supply and input/output (I/O) section included in the presently preferred system of the present invention.

Figure 11 is a graph showing variation in oxygen saturation as a function of hematocrit.

Figure 12 is a graph of $\varepsilon_{b805}/\varepsilon_{b970}$ versus Hematocrit.

Figures 13A-13B are graphs of $\in$ versus Hematocrit at two non-preferred wavelengths and $\varepsilon_1/\varepsilon_2$ versus Hematocrit

at those non-preferred wavelengths.

Figures 14A-14B are graphs of ∈ versus Hematocrit at two non-preferred wavelengths and $\varepsilon_1/\varepsilon_2$ versus Hematocrit at those non-preferred wavelengths.

Figure 15 illustrate vertical emitter alignment and the resulting non-identical $\Delta X_b$ regions.

Figure 16 illustrates horizontal emitter alignment.

## DETAILED DESCRIPTION OF THE PREFERRED EMBODIMENTS

The present invention is directed to apparatus and methods for determining the hematocrit value, transcutaneously and noninvasively. This is achieved by passing at least two wavelengths of light onto or through body tissues such as the finger, earlobe, or scalp, etc., and then compensating for the effects of body tissue and fluid by modifying the Beer-Lambert Law. The principles within the scope of the present invention may also be utilized to provide a hematocrit-independent oxygen saturation and oxygen content measurements as well as noninvasive measurement of blood constituents such as glucose, cholesterol, bilirubin, creatinine, etc.

Although the present description will describe in great detail the transillumination of various body parts, it will be appreciated that reflectance spectrophotometry may alternatively be employed where transillumination is difficult to accomplish. As used herein, the term "body part" is intended to include skin, earlobe, finger, lip, forehead, etc. Because the principles within the scope of the present invention can be adapted by those skilled in the art for in vitro measurement of hematocrit and other blood constituents, the term "body part" is also intended to include various in vitro blood containers such as tubes and cuvettes.

### 1. Spectrophotometric Methods

Spectrophotometric methods have been described in the prior art which monitor various metabolites in body fluids. Radiation, typically in the visible or near infrared region, is directed onto an exterior body part for transcutaneous penetration of the radiation. The radiation is then monitored reflectively or transmissively by a photodetector or similar sensor. Radiation spectra are chosen at wavelengths where the metabolite or compound sought for either absorbs highly or poorly. Some examples of such spectrophotometric methods are described in U.S. Patent No. 4,653,498 for pulse oximetry, U.S. Patent No. 4,655,225 for blood glucose monitoring, and more recently U.S. Patent No. 4,805,623 for monitoring various blood metabolites (glucose, cholesterol, etc.).

A theoretical basis for the spectrophotometric techniques is the Beer-Lambert Law:

$$I = I_0 \ell^{-\varepsilon X d} \qquad (1)$$

Equation (1) may also be written:

$$\ln(I/I_0) = -\varepsilon X d \qquad (1a)$$

wherein $I_0$ is the incident intensity of the source radiation, I is the transmitted intensity of the source through the sample, ε is the extinction coefficient of the sought for component, X is the concentration of the sample component in the tissue itself, and d is the optical path length (distance).

The Beer-Lambert Law (1) permits in vitro solute concentration determinations. However, quantitative measurements have not been possible in the body since the scattering of the incident photons passing into and through the integument and subdermal regions is extensive and highly variable. This scattering spoils the Beer-Lambert Law by adding a variable loss of radiation to the measurement and also extends the path length of the incident photons by an unknown amount as well.

Even though optical pulse rate monitors, plethysmographs, and pulse oximeters are known, their development has been accelerated by techniques which allow for cancellation of the optical scattering effects to a large extent. This development began with U.S. Patent No. 2,706,927 and was further refined by Yoshiya, et. al. (Med. and Biol. Eng. and Computing, 1980 Vol. 18, Pages. 27-32), Koneshi in U.S. Patent No. 3,998,550, and Hamaguri in U.S. No. Patent 4,266,554, which utilized a technique of analyzing the resultant opto-electronic signal by dividing it into its AC and DC components. The AC and DC components are manipulated with logarithmic amplifiers in such a way as to eliminate the above-mentioned transdermal optical effects (the variable amount of radiation loss due to scattering in the tissue and the unknown and variable amounts of optical path length increase).

A paper "Measurement of blood hematocrit by dual-wavelength near-IR photoplethysmography", SPIE Vol,

1641 (1992) pp 150 - 161, JM Schmitt et al describe experiments to test the feasibility of a non-invasive method for measuring arterial blood hematocrit. It is suggested that hematocrit is related to the ratios of the pulsatile and non-pulsatile components of radiation at different wavelengths after transmission through blood-perfused tissue.

Until now, the AC-DC cancellation techniques have not been successfully adapted for the measurement of hematocrit or hematocrit-independent blood oxygen saturation.

2. Noninvasive Differential-Ratiometric Spectrophotometry

It is assumed that incident radiation passing onto or into a living tissue will pass through a combination of blood, tissue, and interstitial fluid compartments. The light attenuated by such a living tissue can be expressed by the modified Beer-Lambert equation:

$$I = I_0 \ell^{-(\varepsilon_b(X_a+X_v)+\varepsilon_t X_t+\varepsilon_i X_i)d+G} \quad (2)$$

Equation (2) may also be written

$$\ln(I/I_0) = -(\varepsilon_b(X_a+X_v)+\varepsilon_t X_t+\varepsilon_i X_i)d+G \quad (2a)$$

Where $\varepsilon_b$, $\varepsilon_t$, and $\varepsilon_i$ represent the extinction coefficient in the blood, tissue, and interstitial fluid compartments, respectively; $X_a$ and $X_v$ represent the arterial and venous blood concentration ($X_b=X_a+X_v$), $X_t$ represents the concentration of the tissue absorbers, and $X_i$ represents the relative concentration of water and dissolved components in the interstitial fluid compartment; d represents the intrasensor spacing; and G is a constant of the geometric configuration.

As the blood layer pulsates, the concentration terms change. The term d can be fixed by the geometric configuration of the device. Taking the partial derivatives of equation (2) with respect to time and dividing by equation (2) gives:

$$-\frac{\partial I/\partial t}{I} = \left(\epsilon_b\left(\partial X_a/\partial t+\partial X_v/\partial t\right)+\epsilon_t \partial X_t/\partial_t+\epsilon_i \partial X_i/\partial t\right)d+\partial G/\partial t \quad (3)$$

which can be simplified at each compartment and wavelength by letting X' =∂X/∂t, and G' =∂G/∂t, and

$$V'_\lambda = -\left(\frac{\partial I/\partial t}{I}\right)_\lambda$$

to give

$$V'_\lambda = \left(\epsilon_b\left(X'_a+X'_v\right)+\epsilon_t X'_t+\epsilon_i X'_i\right)d+G' \quad (4)$$

Assuming that $X_t$ and G do not vary significantly over the pulse time interval, then G'=0 and $X'_t=0$, and equation (4) can be simplified to

$$V'_\lambda = \left(\epsilon_b\left(X'_a+X'_v\right)+\epsilon_i X'_i\right)d \quad (5)$$

Examining the transport between $X_a$ and $X_v$, we can form a proportionality constant $K_v$ such that $X'_v=-K_v X'_a$, representing the reactionary nature of the venous component, and further reduce the above equation to

$$V'_\lambda = \left(\epsilon_b (1 - K_v) X'_a + \epsilon_i X'_i\right) d \qquad (6)$$

Since $X'_a$ and $X'_i$ are not wavelength ($\lambda$) dependent, $V'_\lambda$ values at different wavelengths can be differentially subtracted to produce a hematocrit independent term which contains only $\varepsilon_i X'_i$ information. Although the term $V'_{805}/V'_{1310}$ provides useful information regarding relative changes in hematocrit, it should be recognized that the simple $V'_{805}/V'_{1310}$ ratio is not sufficiently accurate for hematocrit value determination unless the $\varepsilon_i X'_i$ term is known or eliminated. For example, the $\varepsilon_{i805} X'_i$ term can be neglected since $\varepsilon_{i805}$ is extremely small, whereas the $\varepsilon_{i1310} X_i$; term is about 25%-50% of the $\varepsilon_{b1310}$ value of blood itself and cannot, therefore, be neglected without affecting accuracy.

Figures 3 and 12 suggest that a linear combination of $V'_\lambda$ at $\lambda$=805 nm and $\lambda$=970 nm will have a near constant value for a range of Hct values. Since the extinction coefficients $\varepsilon_{i805}$ and $\varepsilon_{i970}$ are well known, or can be empirically determined, a precise proportionality constant $R_1$ can be found to produce

$$\varepsilon_{i970} X'_i = V'_{970} - R_1 V'_{805} \qquad (7)$$

This correction term can now be applied with a second proportionality constant $R_2$ (where $R_2$ is approximately equal to $\varepsilon_{i1310}/\varepsilon_{i970}$) to the $V'_{1310}$ term to exactly remove its $\varepsilon_{i1310} X'_i$ sensitivity, hence:

$$\epsilon_{b1310} (1 - K_v) X'_a = V'_{1310} - R_2 \left(V'_{970} - R_1 V'_{805}\right) \qquad (8)$$

This corrected term can now be used ratiometrically with $V'_{805}$ to remove the $(1-K_v)X'_a$ and leave the pure extinction coefficient ratio represented by Equation (9) below and shown graphically in Figure 4.

$$\frac{\epsilon_{b805}}{\epsilon_{b1310}} = \frac{V'_{805}}{V'_{1310} - R_2 \left(V'_{970} - R_1 V'_{805}\right)} \qquad (9)$$

It should be noticed that the following assumptions and requirements are essential in hematocrit determinations (but in the case of pulse oximetry these requirements may not be of the same degree of significance).

A. Even though wavelengths $\lambda$=805 nm and $\lambda$=1310 nm are near isobestic, the actual function of $\varepsilon$ versus Hematocrit at each given wavelength must hold hematocrit information that is different in curvature, or offset, or linearity, or sign from the other. See Figure 3. If the functions $\varepsilon_\lambda$ versus hematocrit are not sufficiently different, then the ratio $\varepsilon_{b\lambda1}/\varepsilon_{b\lambda2}$ will not hold hematocrit information. See Figures 13A and 13B and Figures 14A and 14B. Even though the foregoing discussion refers to the isobestic wavelengths of $\lambda$=805 nm and $\lambda$=1310 nm, it will be appreciated that other isobestic wavelengths, such as $\lambda$=570 nm, $\lambda$=589 nm, and $\lambda$=1550 nm, may also be utilized.

B. Further, the wavelengths should be selected close enough to one another such that the optical path lengths, d, are approximately the same. Longer wavelengths are preferred since they exhibit less sensitivity to scattering, s:

$$s \propto \frac{1}{\lambda^2} \qquad (10)$$

C. The geometric or spatial relationship of the emitters and sensors is important. For instance, if vertically aligned emitters are used in an earlobe-measuring device, then the top-most emitter may illuminate a different amount of blood filled tissue than the lower emitter. If only one sensor is used, then there will be a disparity between $X'_b$ at each wavelength. See Figure 15, wherein $X_{b1} > X_{b2} > X_{b3}$. Furthermore, the sensor-emitter spatial separation distance is very important because the pressure applied to the tissue between the sensor and emitters affects the arteriolar and capillary vessel compliance. This changes the X' as the pressure (or distance) changes. This change

in X' then modulates the $V'_\lambda$ function. Therefore, the sensor-emitter separation distance must be such that the pressure applied to the earlobe, fingertip, or other body member, does not affect the $V'_\lambda$ function. This sensor separation distance is empirically determined and should generate less than 40 mm Hg applied transmural pressure.

A horizontal alignment (Figure 15) of the emitters with respect to the single sensor can be arranged so that the emitters and sensors illuminate and detect identical regions of $\partial X_{\lambda 1}$ and $\partial X_{\lambda 2}$. It is important to note that the term d, the sensor-emitter separation, will be different between $\lambda_1$ and $\lambda_2$ by the cosine of the angle between the sensor and emitter. Therefore, if any misalignment from normal occurs, the term d will not cancel to obtain equation (9).

The preferred arrangement is wherein all the emitters (660, 805, 950, and 1310 nm) are located on the same substrate. This is preferred because the emitters will then illuminate essentially the same $X_b$ region.

D. In the case of reflectance spectrophotometry, an aperture for the sensor and each emitter is required. See Figure 1B. Also, a sensor-emitter separation is required so that the reflectance of the first layer of tissue, $R_t$, (a non-blood layer of epithelium) does not further exaggerate a multiple scattering effect, i.e. the total reflectance, R, measured would contain spurious information of the epithelial layers' reflectance as well, where:

$$R = R_t + \frac{T_t^2 \cdot R_b}{(1 - R_b \cdot R_t)} \qquad (11)$$

where R is the total reflectance, $R_t$ is the reflectance due to the first tissue-epithelial layer, $R_b$ is the reflectance due to the blood layer, and $T_t$ is the transmission through the first tissue layer.

The reflectance equations describing $R_t$ or $R_b$ must now sum all of the backscattered light that the sensor detects, i.e.,:

$$R_b = \int\int(\text{source function})\cdot(\text{scattering function}) \qquad (12)$$

While equation (9) describes the theory of the noninvasive hematocrit device, the four assumptions (A-D) are important to the repeatability and accurate functioning of the hematocrit device.

Assuming items A through D are dealt with appropriately, then (9) becomes:

$$\frac{\varepsilon_{b\lambda 1}}{\varepsilon_{b\lambda 2}} = \frac{(s_1 + k_1)}{(s_2 + k_2)} \qquad (13)$$

where s is a scattering constant and k is an absorption constant, and where in whole blood:

$$s = \sigma_s Hct(1-Hct) \qquad (14)$$

$$k = \sigma_a Hct \text{ (at isobestic wavelengths)} \qquad (15)$$

where $\sigma_s$ is the scattering cross section and $\sigma_a$ is the absorption cross section.

From the foregoing, ε, the extinction coefficient, is not a simple function of the absorption coefficient, k, normally determined in pure solutions. Rather, it contains a diffusion or scattering term, s, which must be accounted for in a non-pure solution media such as whole blood and tissue.

Finally, substituting (14) and (15) into (13):

$$\frac{\varepsilon_{b\lambda 1}}{\varepsilon_{b\lambda 2}} = \frac{\sigma_{s1}(1-Hct) + \sigma_{a1}}{\sigma_{s2}(1-Hct) + \sigma_{a2}} \qquad (16)$$

Therefore, the ratio $\varepsilon_{\lambda 1}/\varepsilon_{\lambda 2}$ is a function of hematocrit. From Figure 4, a look up table or polynomial curve fit equation may be obtained and utilized in the final displayed hematocrit results. Knowing the actual hematocrit value, it is straightforward to see (Figure 2) that a wavelength at 660 nanometers can be selected to obtain an ε ratio wherein the hematocrit-independent oxygen saturation value is derived. For example, equation (16) would become:

$$\frac{\varepsilon_{b660}}{\varepsilon_{b805}} = \frac{\sigma_{s660}(1\text{-Hct}) + \sigma_{a660} + S_aO_2(\sigma_{ao660} - \sigma_{ar660})}{\sigma_{s805}(1 - \text{Hct}) + \sigma_{a805} + S_aO_2(\sigma_{a0805} - \sigma_{as805})} \quad (17)$$

Equation (17) shows both the hematocrit and oxygen saturation dependence on each other.

Figure 11 graphically demonstrates the need for a hematocrit-independent blood saturation device. As either the hematocrit value or percent oxygen saturation decreases, the percent saturation error becomes unacceptable for clinical usage. For example, it is not uncommon to see patients with a low hematocrit (about 20%) who have respiratory embarrassment (low oxygen saturation) as well. Hence, the clinician simply requires more accurate oxygen saturation values.

Knowing the hematocrit and oxygen saturation values, the computation of the Oxygen Content is trivial and may be displayed directly (a value heretofore unavailable to the clinician as a continuous, real-time, noninvasive result):

$$[\text{Oxygen Content}] = \text{Hct} \bullet S_aO_2 \bullet K \quad (18)$$

where K is an empirically determined constant.

Referring to the equations (16) and (9) a decision must be made by the computer as to the suitability of utilizing the Taylor expansion approximation to the logarithm. This algorithm is maintained in the software as a qualifying decision for the averaging and readout algorithms. The Taylor approximation is only valid for small $\partial I/\partial t$ values.

3. Nonpulsatile Applications

a. Valsalva's Maneuver to Simulate Pulsatile Case

It is interesting to see the similarities between this AC pulsatile derivation and an analogous DC technique. By taking the logarithm of two intensity ratios, values of $\varepsilon_b$ and $\varepsilon_i$ can be obtained from the modified Beer-Lambert equation (equation (2a)). These same extinction coefficients can be manipulated by the identical proportionality constants $R_1$ and $R_2$ found previously to exactly eliminate $\varepsilon_{i1310}X_i$ and yield

$$\frac{\epsilon_{b805}}{\epsilon_{b1310}} = \frac{U_{805}}{U_{1310} - R_2(U_{970} - R_1 U_{805})} \quad (19)$$

Where the term

$$U_\lambda = \ln\left(\frac{I_2}{I_1}\right)_\lambda$$

represents the logarithm of the ratio of intensities at $X_b$ values of $X_{b1}$ and $X_{b2}$.

It should also be noted that the two derivations (AC and DC) fold into' one another through the power series expansion of the ln(1+Z) function:

$$\ln(1+Z) = Z - \frac{Z^2}{2} + \frac{Z^3}{3} - \cdots \quad (20)$$

When the value $\Delta I = I_2 - I_1$, it can be seen that

$$\ln\left(\frac{I_2}{I_1}\right) = \ln\left(\frac{\Delta I + I_1}{I_1}\right) = \ln\left(1 + \frac{\Delta I}{I_1}\right) = \frac{\Delta I}{I_1} + \text{High Order Terms} \quad (21)$$

which means that for small changes in $X_b$, the AC (partial derivative) and DC (logarithmic) derivations are similar and can each be precisely compensated through this differential-ratiometric technique to provide an noninvasive $\varepsilon_{b805}/\varepsilon_{b1310}$ ratio which is independent of both the constant and time-varying tissue and interstitial fluid terms.

One currently preferred method of obtaining the two intensities is to have the patient perform Valsalva's maneuver. Valsalva's maneuver is an attempt to forcibly exhale with the glottis, nose, and mouth closed. This maneuver increases intrathoracic pressure, slows the pulse, decreases return of blood to the heart, and increases venous pressure. Obtaining intensity measurements before and during Valsalva's maneuver provide sufficiently different intensities to utilize equation (19). Even a deep breath can be enough to obtain sufficiently different intensities

b. Stepper Motor Technique

Another technique to simulate pulsatile blood flow and to eliminate the skin's optical scattering effects, while at the same time preserving the blood-borne hematocrit and oxygen saturation information, is described below. By utilizing a stepper motor 9 in the earlobe clip assembly 10 on an earlobe 11 of a patient such as that illustrated in Figures 6, 6A, 15, and 16, one can produce a variation of $X_b$ sufficient to utilize equation 19. The stepper motor 9 could even produce a bloodless ($X_b$=0) state, if required. However, equation 19 shows that only a difference between $X_{b1}$ and $X_{b2}$ is needed.

The major advantage of this technique is that under clinical conditions of poor blood flow, poor blood pressure, or peripheral vascular disease, where pulse wave forms are of poor quality for the $(\partial I/\partial t)/I$ technique, this DC stepper motor technique could be utilized.

c. Oxygen Saturation Determination

The above techniques describe conditions and equations wherein isobestic wavelengths are chosen such that the hematocrit value obtained has no interference from oxygen saturation, hence an independently determined hematocrit value.

One, however, may choose $\lambda_2$ (the reference wavelength) in equation (13) at 1550 nm as well. In the radiation region 900 to 2000 nm the blood absorption coefficients depend on hematocrit and water, whereas at 805 nm the blood absorption coefficient only depends on hematocrit. Therefore, utilizing in combination, wavelengths of 660, 805, and 1550 will also give a technique to determine hematocrit ($\varepsilon_{805}/\varepsilon_{1550}$) and oxygen saturation ($\varepsilon_{660}/\varepsilon_{805}$).

These 3 wavelengths are particularly important since 660, 805, and 1550 nm (or 1310 nm) are readily available LEDs, such as, respectively, MLED76-Motorola, HLP30RGB-Hitachi, and ETX1550-EPITAXX (or NDL5300-NEC), with the benefits of low cost and low optical power (reducing any question of possible eye damage).

The manufacturing of a multi-chip LED emitter becomes reasonable, cost-wise, and provides increased accuracy since the LED sources have practically no separation distances and appear as a single point source.

The described techniques may be applied to the determination of other components (included, but not limited to, glucose, or cholesterol) in any range of the electromagnetic spectrum in which spectrophotometric techniques can be utilized.

4. Currently Preferred Apparatus

An earlobe clip assembly 10 as in Figures 6, 6A, 15, and 16 (with or without the stepper motor 9 shown in Figure 6A) a finger clip assembly 6 used on a finger 7 as shown in Figures 1, 1A, and 1B are two currently preferred embodiments for practicing the present invention. The photodiodes 3 and emitters 1 and 2 in each are placed in accordance with appropriate alignment.

Consider first the sensor technology in the transmissive mode of operation. An earlobe or fingertip housing can be provided with discreet emitters and two photodiode chips (of different sensitivity ranges, 600-1000 nm and 1000-1700 nm ranges) placed on one substrate, such as a TO-5 can (Hamamatsu K1713-03). The emitters likewise can be two or more emitter chips (i.e., $\lambda$ = 805, 1310, 660, and 950 nm) placed on a common substrate and illuminated through a TO-39 can.

Finally, a single substrate multi-wavelength emitter and a multi-wavelength detector, assembled in one small physical housing for each, make alignment and detection sensitivity more repeatable, and hence more accurate.

The preferred emitter chips would have wavelengths, for hematocrit-only measurements, at 805 nm, 950 nm,

and 1310 nm (or 805 nm, 950 nm, and 1550 nm). Although in theory, an emitter having a wavelength of 970 nanometers, rather than 950 nm, would provide more accurate information, 970 nm emitters are not presently available commercially. These wavelengths are currently preferred because of the different curvature and baseline offset of the ε versus Hematocrit at those wavelengths. See Figure 3. Hence, the hematocrit information will exist in the ratio $\varepsilon_{\lambda 1}/\varepsilon_{\lambda 2}$. See Figure 4.

Furthermore, the choice of 805 nm and 1310 nm (or 1550 nm) rather than 570 nm and 805 nm is because there is no water absorption in the 570 nm (or 589 nm) and 805 nm isobestic wavelengths. However, there is tremendous water absorption at 1310 nm and 1550 nm. Hence, the ratio of 570 nm to 805 nm, as a reference, would not yield hematocrit information because there would be no offset due to water in the plasma. See Figures 13A and 13B and Figures 14A and 14B.

If hematocrit-independent oxygen saturation is desired then the emitter chip wavelengths would be 660 nm, 805 nm, 950 nm, and 1310 nm (or 1550 nm) (the 660 nm is MLED76, Motorola or TOLD 9200, Toshiba). Likewise, the photodetector single substrate could house at least two chips, such as a Hamamatsu K1713-03.

It will be appreciated that those skilled in the art would be able to add other chips to the single substrate at wavelengths sensitive to other metabolites (glucose, cholesterol, etc.). The above mentioned emitter and detector connections can be seen in the analog schematic diagram illustrated in Figures 7 and 9B-9D.

The sensor technology in the reflectance mode must conform to two embodiment parameters. See Figure 1B. The diameter and thickness of the aperture 8 in which figure 7 is received in combination with the sensor-emitter separation distance are important to provide a detection region within the subdermis 12 at points a and b of Figure 1B, where the radiation impinges on blood-tissue without the multiple scattering effects of the epithelia layer, $R_t$. The determination of optimum sensor 3 separatio and aperture 8 sizes is done empirically from numerou fingers 7 with varying callous and fingernails 13. Minimum sensor separation and aperture diameters can be establishe wherein $R_t$, of equation (14) is eliminated.

Figures 7, 8A-8C, 9A-9D, and 10A-10B detail th electronics of one circuit suitable for use within th scope of the present invention. The memory and computatio: means (Figures 8A-8C) are connected via a "bus" structur between PROMS (U110,U111), microprocessor MC68HC000 (U106) static RAMS (U112,U113), and isolation buffers to the low-level analog circuitry (Figure 7). A crystal controlled oscillator circuit (U101A,B) is divided by 2 to provides a symmetric master clock to the microprocessor; this clock is further subdivided and used to provide clocking for the analog-to-digital converter (U208) and timer (U109) Strobe lines are generated through a decoder arrangement to drive each of the subsystems of the device and also control the isolation bus buffers (U201,U202).

Timer outputs are fed back into the microprocessor and encoded (U104) to produce interrupts at specific intervals for system functions. One timer is shared by subsystems which control the liquid crystal display means, the keyboard entry means, the audible indicator, and the cycling background system self-test. Another timer is dedicated exclusively to provide a high priority interrupt to the microprocessor; this interrupt drives software which controls the basic sensor sampling mechanism. An expansion connector (J101) is included to allow extended testing of the device or connection to external data-logging equipment such as a printer or computer interface.

The local bus isolates the sensitive analog circuitry from the main digital circuitry. This prevents spurious crosstalk from digital signals into the analog circuitry and thereby reduces superimposed noise on the measured signals. It is on this local bus that the Digital-to-Analog Converters (DAC) and Analog-to-Digital Convertors (ADC) transmit and receive digital information while processing the low-level analog signals.

The Low Level Sensor electronic section, Figure 7, combines subsystems to both measure and modulate the current produced from each optical sensor. Since the pulsatile component of the optical energy transmitted through or reflected off of tissue comprises only a small part of the overall optical energy incident on the sensor, means are provided to "null out" in a carefully controlled and accurately known way the non-pulsatile component of the light-produced current in the sensing detector. The remaining signal can then be dc-amplified and filtered in a straightforward manner and presented to the ADC (U208) for conversion into a digital value representative of the relative AC pulsatile component. Furthermore, because the relationship between the nulling current and the average value of this AC component is known, the DC component can easily be calculated as a function of the sensing means' sensitivities and the electronic stages' gains. The functions determining these AC and DC values can (if necessary) be trimmed in software by calibration constants which are stored in EEPROM (U307) and retrieved each time the unit is powered on.

The current which modulates the optical sources (LEDs or Laser Diodes) is also controlled (U203) and precisely adjusted (U306) to optimize signal reception and detection. Through software control, the modulation current can be adjusted on a pulse-by-pulse basis to minimize noise-induced inaccuracies. Furthermore, by sampling the sensors with the modulation sources disabled appropriately, background noise (such as 60 Hz) can be rejected digitally as common-mode noise. Thus, by controlling the optical source energy and modulating the nulling current in the photosensor circuitry, it is possible to effectively cancel the effects of ambient radiation levels and accurately measure both the static (DC) and time-varying (AC) components of transmitted or reflected light.

Interrupt-driven software algorithms acquire the sensor data, provide a real-time pulse wave contour, and

determine pulse boundaries. Completed buffers (i.e. one entire pulse per buffer) of sensor data are then passed to the foreground software processes for computation. This involves the determination of the background-compensated AC pulsatile and DC static values of intensities for each wavelength. Through averaging and selective elimination of abnormal values, results are then calculated using equation (9) and displayed on the LCD. The modulating and nulling currents are (if necessary) also adjusted to utilize the electronic hardware efficiently and optimally.

5. Summary

Although the foregoing discussion has related to noninvasive analysis of blood hematocrit information, it will be appreciated that the above-mentioned emitters, sensors, and circuitry may be adapted for invasive in vitro analysis of blood hematocrit values. The principles within the scope of the present invention which compensate for spatial, geometric, and tissue variations may be used to compensate for similar variations in an in vitro blood container. Such a device would allow hematocrit values to be determined rapidly and accurately.

Those skilled in the art will also appreciate that the methods for determining blood hematocrit values may be adapted for determining non-hematocrit biologic constituent values such as glucose, cholesterol, etc. To determine biologic constituent information, the effects of competing blood, tissue, and interstitial fluid constituents must be eliminated. It is believed that these effects may be eliminated by appropriate modification of the differential ratiometric techniques described above.

It is important to recognize that the present invention is not directed to determining the tissue hematocrit value. The tissue hematocrit value, in contrast with the blood hematocrit value, reflects the amount of red blood cells in a given volume of tissue (blood, interstitial fluids, fat, hair follicles, etc.). The present invention is capable of determining actual intravascular blood hematocrit values.

From the foregoing, it will be appreciated that the present invention provides a system and method for noninvasively and quantitatively determining a subject's hematocrit and optionally other blood constituent value. The present invention determines the hematocrit noninvasively by utilizing electromagnetic radiation as the transcutaneous information carrier. Importantly, the present invention may be used on various body parts to provide accurate quantitative hematocrit values.

It will also be appreciated that embodiments of the present invention also provides a system and method which can provide immediate and continuous hematocrit information for a subject. There is also disclosed a system and method for noninvasively determining a subjects's blood oxygen saturation ($S_aO_2$) independent of the subject's hematocrit. In addition, there is also disclosed a system and method for noninvasively determining a subject's hematocrit and/or blood oxygen saturation even under conditions of low blood perfusion.

The present invention may be embodied in other specific forms without departing from its essential characteristics. The described embodiments are to be considered in all respects only as illustrative and not restrictive. The scope of the invention is, therefore, indicated by the appended claims rather than by the foregoing description.

## Claims

1. A method for determining hematocrit of the blood of a patient, the blood flowing in a body part (7) of the patient or in an extracorporeal passageway in communication with the circulatory system of the patient so as to be subjectable to transcutaneous examination in the extracorporeal passageway, the body part or extracorporeal passageway defining a blood conduit, the method comprising the steps of:

   selecting a first radiation wavelength having a first extinction characteristic as a function of hematocrit;
   selecting a second radiation wavelength which exhibits a greater absorption coefficient to water than said first radiation wavelength, said second radiation having a second extinction characteristic as a function of hematocrit different from the first extinction characteristic in at least one of curvature, offset, linearity and sign;
   directing (1)(2) said first and second radiation wavelengths into the blood conduit;
   detecting (3) the amount of first radiation after passing through the blood conduit;
   detecting (3) the amount of second radiation after passing through the blood conduit; and
   comparing variations over time in the detected amounts of first and second radiations to determine the hematocrit value by a differential ratiometric technique, whereby said hematocrit value is determined quantitatively .

2. A method as claimed in claim 1 wherein the first and second wavelengths are selected at the isobestic points of reduced haemoglobin and oxyhaemoglobin.

3. A method as claimed in claim 1 or 2 wherein said first and second extinction characteristics comprise first and

second curvatures.

**4.** A method as claimed in claim 1 or 2 wherein said first and second extinction characteristics comprise first and second offsets.

**5.** A method as claimed in claim 1 or 2 wherein said first and second extinction characteristics comprise first and second linearity characteristics.

**6.** A method as claimed in claim 1 or 2 wherein said first sign is different from said second sign.

**7.** A method as claimed in any preceding claim, further comprising the steps of:

selecting a third radiation wavelength which will be extinguished by a competing biologic constituent in the blood in a manner characteristic of said competing biologic constituent and having an extinction characteristic with regard to constituents other than said competing biologic constituent which is substantially different from one of said first and second extinction characteristics;
directing radiation having the third wavelength into the blood conduit;
determining the amount of radiation having the third wavelength which is extinguished by the blood conduit;
operating on the amount of detected radiation having the first, second and third wavelengths such that the spatial, geometric and tissue variations are eliminated in each radiation wavelength; and
compensating for the effect of the second biologic constituent by operating on the amount of detected radiation having first, second and third wavelengths.

**8.** A method as claimed in any preceding claim wherein said comparison step comprises mathematically manipulating the detected amount of first and second radiation wavelengths with a polynomial function to determine the hematocrit value.

**9.** A method as claimed in any preceding claim wherein said comparison step comprises forming the ratio of the $\Delta I/I$ ratios for each of the first and second radiation wavelengths, where $\Delta I$ represents a time derivative of a pulsatile transmitted intensity component, and I represents a steady state transmitted intensity.

**10.** A system for determining hematocrit as a biological constituent value of the blood of a patient, the blood flowing in a body part of the patient or in an extracorporeal passageway in communication with the circulatory system of the patient so as to be subjectable to transcutaneous examination in the body part or to non-invasive examination in the extracorporeal passageway, the body part (7) or the extracorporeal passageway defining a blood conduit, the system comprising:

blood conduit receiving means (6) for receiving a blood conduit containing the flowing blood of the patient;
a first emitter (1) positioned on said conduit receiving means for emitting a first radiation wavelength, said first radiation having at least a first extinction characteristic with regard to hematocrit;
a second emitter (2) positioned on said conduit receiving means for emitting a second radiation wavelength which exhibits a greater absorption coefficient to water than said first radiation wavelength, said second radiation having at least a second extinction characteristic different from the first extinction characteristic in at least one of curvature, offset, linearity or sign;
directing means for directing said first and second radiation wavelengths into the blood conduit;
first detecting means (3) for detecting the amount of first radiation after passing through the blood conduit;
second detecting means (3) for detecting the amount of second radiation after passing through the blood conduit; and
means for comparing variations over time in the detected amounts of first and second radiations to determine the hematocrit value by a differential ratiometric technique, whereby said hematocrit value is determined quantitatively.

**11.** A system as claimed in claim 10, wherein said first and second wavelengths are at or near the isobestic points of reduced haemoglobin and oxyhaemoglobin.

**12.** A system as claimed in claim 10 or 11 wherein said first and second extinction characteristics comprise first and second curvatures.

13. A system as claimed in claim 10 or 11 wherein said first and second extinction characteristics comprise first and second offsets.

14. A system as claimed in claim 10 or 11 wherein said first and second extinction characteristics comprise first and second linearity characteristics.

15. A system as claimed in claim 10 or 11 wherein said first sign is different from said second sign.

16. A system as claimed in any of claims 10 to 15 wherein said first and second photodetecting means are formed by a single photodetector.

17. A system as claimed in any of claims 10 to 16, the system further comprising:

    a third emitter positioned on said conduit receiving means for emitting a third radiation wavelength which will be extinguished by a competing biologic constituent in the blood in a manner characteristic of said competing biologic constituent and having an extinction characteristic with regard to constituents other than said competing biologic constituent which is substantially different from one of said first and second extinction characteristics;
    second directing means for directing radiation having the third wavelength into the blood conduit;
    detecting means for detecting the amount of radiation having the third wavelength which is extinguished by the blood in the blood conduit;
    means for operating on the amount of detected radiation having the first, second and third wavelengths such that the spatial, geometric and tissue variations are eliminated in each radiation wavelength; and
    means for compensating for the effect of the second biologic constituent by operating on the amount of detected radiation having first, second and third wavelengths.

18. A system as claimed in any of claims 10 to 17 wherein said comparing means is arranged for mathematically manipulating the detected amount of first and second radiation wavelengths with a polynomial function to determine the hematocrit value.

19. A system as claimed in any of claims 10 to 18, wherein said comparing means comprises means for forming the ratio of the $\Delta I/I$ ratios for each of the first and second radiation wavelengths, where $\Delta I$ represents a time derivative of a pulsatile transmitted intensity component, and I represents a steady state transmitted intensity.

**Patentansprüche**

1. Verfahren zur Bestimmung des Hämatokrit des Bluts eines Patienten, wobei das Blut in einem Körperteil (7) des Patienten oder in einem extrakorporalen Durchgang fließt, der an das Kreislaufsystem des Patienten angeschlossen ist, um auf diese Weise einer transkutanen Untersuchung in dem extrakorporalen Durchgang unterzogen werden zu können, wobei das Körperteil oder der extrakorporale Durchgang einen Blutkanal bildet, wobei das Verfahren die Schritte umfasst:

    Auswahl einer ersten Strahlungs-Wellenlänge mit einer ersten Extinktions-Charakteristik als Funktion des Hämatokrit;
    Auswahl einer zweiten Strahlungs-Wellenlänge, die einen größeren Absorptionskoeffizienten bezüglich Wasser aufweist als die erste Strahlungs-Wellenlänge, wobei die zweite Strahlung eine zweite Extinktions-Charakteristik als Funktion des Hämatokrit hat, die sich von der ersten Extinktions-Charakteristik in wenigstens einem aus Krümmung, Offset, Linearität und Vorzeichen unterscheidet;
    Einleiten (1) (2) der ersten und zweiten Strahlungs-Wellenlänge in den Blutkanal;
    Erfassen (3) der Menge an erster Strahlung nach dem Durchgang durch den Blutkanal;
    Erfassen (3) der Menge an zweiter Strahlung nach dem Durchgang durch den Blutkanal; und
    Vergleichen der zeitlichen Veränderungen der erfassten Mengen an erster und zweiter Strahlung, um den Hämatokritwert durch eine Differentialratiometer-Technik zu bestimmen, wobei der Hämatokritwert quantitativ bestimmt wird.

2. Verfahren nach Anspruch 1, worin die erste und die zweite Wellenlänge an den isobestischen Punkten von reduziertem Hämoglobin und Oxyhämoglobin gewählt werden.

**3.** Verfahren nach Anspruch 1 oder 2, worin die erste und zweite Extinktions-Charakteristik erste und zweite Krümmungen aufweisen.

**4.** Verfahren nach Anspruch 1 oder 2, worin die erste und zweite Extinktions-Charakteristik erste und zweite Offsets aufweisen.

**5.** Verfahren nach Anspruch 1 oder 2, worin die erste und zweite Extinktions-Charakteristik erste und zweite Linearitätseigenschaften aufweisen.

**6.** Verfahren nach Anspruch 1 oder 2, worin das erste Vorzeichen sich von dem zweiten Vorzeichen unterscheidet.

**7.** Verfahren nach einem der vorangehenden Ansprüche, weiter umfassend die Schritte:

Auswahl einer dritten Strahlungs-Wellenlänge, die von einem konkurrierenden, biologischen Bestandteil im Blut auf eine Weise abgeschwächt wird, die für den konkurrierenden, biologischen Bestandteil charakteristisch ist, und die bezüglich der sich von dem konkurrierenden, biologischen Bestandteil unterscheidenden Bestandteile eine Extinktions-Charakteristik aufweist, die sich von einer der ersten und der zweiten Extinktions-Charakteristik deutlich unterscheidet;
Einleiten von Strahlung mit der dritten Wellenlänge in den Blutkanal;
Erfassen der Strahlungsmenge mit der dritten Wellenlänge, die von dem Blutkanal abgeschwächt wird;
Bearbeiten der Menge an erfasster Strahlung mit der ersten, zweiten und dritten Wellenlänge, so dass die Raum-, Geometrie- und Gewebe-Variationen bei jeder Strahlungs-Wellenlänge beseitigt werden; und
Kompensation des Effekts des zweiten biologischen Bestandteils durch Bearbeiten der Menge an erfasster Strahlung mit der ersten, zweiten und dritten Wellenlänge.

**8.** Verfahren nach einem der vorangehenden Ansprüche, worin der Vergleichsschritt zur Erfassung des Hämatokritwerts eine mathematische Manipulation der erfassten Menge an erster und zweiter Strahlungs-Wellenlänge mit einer Polynomfunktion umfasst.

**9.** Verfahren nach einem der vorangehenden Ansprüche, worin der Vergleichsschritt die Bildung des Verhältnisses der $\Delta I/I$-Verhältnisse für jeweils die erste und die zweite Strahlungs-Wellenlänge umfasst, wobei $\Delta I$ eine zeitliche Ableitung einer in Pulsen übertragenen Komponente der Intensität und I eine stationär übertragene Intensität darstellen.

**10.** System zur Bestimmung des Hämatokrit als biologischen Bestandteilswert des Blut eines Patienten, wobei das Blut in einem Körperteil des Patienten oder in einem extrakorporalen Durchgang fließt, der an das Kreislaufsystem des Patienten angeschlossen ist, um auf diese Weise einer transkutanen Untersuchung in dem Körperteil oder einer nicht-invasiven Untersuchung in dem extrakorporalen Durchgang unterzogen werden zu können, wobei das Körperteil (7) oder der extrakorporale Durchgang einen Blutkanal bildet, wobei das System umfasst:

ein Blutkanalaufnahmemittel (6) zur Aufnahme eines Blutkanals, der das fließende Blut des Patienten enthält;
einen ersten Emitter (1), der an das Kanalaufnahmemittel zum Aussenden einer ersten Strahlungs-Wellenlänge angeordnet ist, wobei die erste Strahlung bezüglich Hämatokrit wenigstens eine erste Extinktions-Charakteristik aufweist;
einen zweiten Emitter (2), der an das Kanalaufnahmemittel zum Aussenden einer zweiten Strahlungs-Wellenlänge, die einen höheren Absorptionskoeffizienten bezüglich Wasser aufweist als die erste Strahlungs-Wellenlänge, angeordnet ist, wobei die zweite Strahlung wenigstens eine zweite Extinktions-Charakteristik aufweist, die sich von der ersten Extinktions-Charakteristik in wenigstens einem aus Krümmung, Offset, Linearität oder Vorzeichen unterscheidet;
ein Einleitmittel zum Einleiten der ersten und zweiten Strahlungs-Wellenlänge in den Blutkanal;
ein erstes Erfassungsmittel (3) zum Erfassen der Menge an erster Strahlung nach dem Durchgang durch den Blutkanal;
ein zweites Erfassungsmittel (3) zum Erfassen der Menge an zweiter Strahlung nach dem Durchgang durch den Blutkanal; und
ein Mittel zum Vergleich der zeitlichen Veränderungen der erfassten Mengen an erster und zweiter Strahlung, um den Hämatokritwert durch eine Differentialradiometer-Technik zu bestimmen, wobei der Hämatokritwert quantitativ bestimmt wird.

**11.** System nach Anspruch 10, worin die erste und die zweite Wellenlänge an den oder nahe der isobestischen Punkte von reduziertem Hämoglobin und Oxyhämoglobin liegen.

**12.** System nach Anspruch 10 und 11, worin die erste und zweite Extinktions-Charakteristik erste und zweite Krümmungen aufweisen.

**13.** System nach Anspruch 10 und 11, worin die erste und zweite Extinktions-Charakteristik erste und zweite Offsets aufweisen.

**14.** System nach Anspruch 10 und 11, worin die erste und zweite Extinktions-Charakteristik erste und zweite Linearitätseigenschaften aufweisen.

**15.** System nach Anspruch 10 und 11, worin das erste Vorzeichen sich von dem zweiten Vorzeichen unterscheidet.

**16.** System nach einem der Ansprüche 10 bis 15, worin die ersten und zweiten Fotoerfassungsmittel aus einem einzelnen Fotodetektor bestehen.

**17.** System nach einem der Ansprüche 10 bis 16, wobei das System weiter umfasst:

einen dritten Emitter, der an den Kanalaufnahmemitteln zum Aussenden einer dritten Strahlungs-Wellenlänge angeordnet ist, die von einem konkurrierenden, biologischen Bestandteil im Blut auf eine Weise abgeschwächt wird, die für den konkurrierenden, biologischen Bestandteil charakteristisch ist, und die bezüglich der sich von dem konkurrierenden, biologischen Bestandteil unterscheidenden Bestandteile eine Extinktions-Charakteristik aufweist, die sich von einer der ersten und zweiten Extinktions-Charakteristik deutlich unterscheidet;
ein zweites Einleitmittel zum Einleiten der Strahlung mit der dritten Wellenlänge in den Blutkanal;
ein Erfassungsmittel zum Erfassen der Menge an Strahlung mit der dritten Wellenlänge, die von dem Blut im Blutkanal abgeschwächt wird;
ein Mittel zum Bearbeiten der Menge an erfasster Strahlung mit der ersten, zweiten und dritten Wellenlänge, so dass die Raum-, Geometrie- und Gewebe-Variationen bei jeder Strahlungs-Wellenlänge beseitigt werden; und
ein Mittel zur Kompensation des Effekts des zweiten biologischen Bestandteils durch Bearbeiten der Menge an erfasster Strahlung mit der ersten, zweiten und dritten Wellenlänge.

**18.** System nach einem der Ansprüche 10 bis 17, worin das Mittel zum Vergleichen zur mathematischen Manipulation zur Erfassung des Hämatokritwerts der erfassten Menge an erster und zweiter Strahlungs-Wellenlänge mit einer Polynomfunktion ausgelegt ist.

**19.** System nach einem der Ansprüche 10 bis 18, worin das Mittel zum Vergleichen ein Mittel zum Bilden des Verhältnisses der $\Delta I/I$-Verhältnisse für jeweils die erste und die zweite Strahlungs-Wellenlänge umfasst, wobei $\Delta I$ eine zeitliche Ableitung einer in Pulsen übertragene Komponente der Intensität und I eine stationär übertragene Intensität darstellen.

## Revendications

**1.** Procédé de détermination du taux d'hématocrite du sang d'un patient, le sang circulant dans une partie du corps (7) du patient ou dans une voie de passage extracorporelle en communication avec l'appareil circulatoire du patient, de façon à être susceptible de faire l'objet d'un examen transcutané dans la voie de passage extracorporelle, la partie du corps ou la voie de passage extracorporelle définissant un conduit de sang, le procédé comprenant les étapes consistant à :

- sélectionner une première longueur d'onde de rayonnement ayant une première caractéristique d'extinction en fonction du taux d'hématocrite ;
- sélectionner une deuxième longueur d'onde de rayonnement présentant un coefficient d'absorption, par rapport à l'eau, supérieur à celui de ladite première longueur d'onde de rayonnement, ledit deuxième rayonnement ayant une deuxième caractéristique d'extinction, en fonction du taux d'hématocrite, différente de la première caractéristique d'extinction par au moins une particularité, parmi la courbe, le décalage, la linéarité et le signe ;
- diriger (1) (2) lesdites première et deuxième longueurs d'ondes de rayonnements jusque dans le conduit de

sang ;

- détecter (3) l'intensité du premier rayonnement après son passage à travers le conduit de sang ;
- détecter (3) l'intensité du deuxième rayonnement après son passage à travers le conduit de sang ; et
- comparer les variations dans le temps des intensités détectées des premier et deuxième rayonnements afin de déterminer la valeur du taux d'hématocrite par une technique de mesure différentielle des proportions, grâce à laquelle ladite valeur du taux d'hématocrite est déterminée de manière quantitative.

**2.** Procédé selon la revendication 1, pour lequel les première et deuxième longueurs d'ondes sont sélectionnées aux points isobestiques de réduction des taux d'hémoglobine et d'oxyhémoglobine.

**3.** Procédé selon la revendication 1 ou 2, pour lequel lesdites première et deuxième caractéristiques d'extinction sont constituées par des première et deuxième courbes.

**4.** Procédé selon la revendication 1 ou 2, pour lequel lesdites première et deuxième caractéristiques d'extinction sont constituées par des premier et deuxième décalages.

**5.** Procédé selon la revendication 1 ou 2, pour lequel lesdites première et deuxième caractéristiques d'extinction sont constituées par des première et deuxième caractéristiques de linéarité.

**6.** Procédé selon la revendication 1 ou 2, pour lequel ledit premier signe est différent dudit deuxième signe.

**7.** Procédé selon l'une quelconque des revendications précédentes, comprenant, en outre, les étapes consistant à :

- sélectionner une troisième longueur d'onde de rayonnement faisant l'objet d'une extinction par un constituant biologique en concurrence dans le sang, d'une manière caractéristique dudit constituant biologique en concurrence, et ayant une caractéristique d'extinction, relativement aux constituants autres que ledit constituant biologique en concurrence, sensiblement différente d'une desdites première et deuxième caractéristiques d'extinction ;
- diriger un rayonnement ayant la troisième longueur d'onde jusque dans le conduit de sang ;
- déterminer l'intensité du rayonnement, ayant la troisième longueur d'onde, faisant l'objet d'une extinction par le conduit de sang ;
- effectuer des opérations sur les intensités des rayonnements détectés, ayant les première, deuxième et troisième longueurs d'ondes, de sorte que les variations spatiales, géométriques et relatives aux tissus soient éliminées pour chaque longueur d'onde de rayonnement ; et
- compenser l'effet du deuxième constituant biologique en effectuant des opérations sur les intensités des rayonnements détectés ayant les première, deuxième et troisième longueurs d'ondes.

**8.** Procédé selon l'une quelconque des revendications précédentes, pour lequel ladite étape de comparaison consiste à manipuler mathématiquement les intensités détectées des première et deuxième longueurs d'ondes de rayonnements avec une fonction polynomiale afin de déterminer la valeur du taux d'hématocrite.

**9.** Procédé selon l'une quelconque des revendications précédentes, pour lequel ladite étape de comparaison consiste à établir le rapport des taux $\Delta I / I$ pour chacune des première et deuxième longueurs d'ondes de rayonnements, $\Delta I$ représentant une dérivée dans le temps d'une composante d'intensité transmise de manière pulsatoire et $I$ représentant une intensité transmise en régime établi.

**10.** Système de détermination du taux d'hématocrite, en tant que valeur d'un constituant biologique du sang d'un patient, le sang circulant dans une partie du corps du patient ou dans une voie de passage extracorporelle en communication avec l'appareil circulatoire du patient, de façon à être susceptible de faire l'objet d'un examen transcutané dans la partie du corps ou d'un examen non-effractif dans la voie de passage extracorporelle, la partie du corps (7) ou la voie de passage extracorporelle définissant un conduit de sang, le système comprenant :

- des moyens de réception de conduit de sang (6) pour recevoir un conduit de sang contenant le sang, en circulation, du patient ;
- un premier émetteur (1), positionné sur lesdits moyens de réception de conduit de sang, pour émettre une première longueur d'onde de rayonnement, ledit premier rayonnement ayant au moins une première caractéristique d'extinction relativement au taux d'hématocrite ;
- un deuxième émetteur (2), positionné sur lesdits moyens de réception de conduit de sang, pour émettre une

deuxième longueur d'onde de rayonnement présentant un coefficient d'absorption, par rapport à l'eau, supérieur à celui de ladite première longueur d'onde de rayonnement, ledit deuxième rayonnement ayant au moins une deuxième caractéristique d'extinction différente de la première caractéristique d'extinction par au moins une particularité, parmi la courbe, le décalage, la linéarité ou le signe ;

- des moyens directeurs pour diriger lesdites première et deuxième longueurs d'ondes de rayonnements jusque dans le conduit de sang ;
- des premiers moyens de détection (3) pour détecter l'intensité du premier rayonnement après son passage à travers le conduit de sang ;
- des deuxièmes moyens de détection (3) pour détecter l'intensité du deuxième rayonnement après son passage à travers le conduit de sang ; et
- des moyens de comparaison des variations dans le temps des intensités détectées des premier et deuxième rayonnements afin de déterminer la valeur du taux d'hématocrite par une technique de mesure différentielle des proportions, grâce à laquelle ladite valeur du taux d'hématocrite est déterminée de manière quantitative.

**11.** Système selon la revendication 10, dans lequel lesdites première et deuxième longueurs d'ondes sont sélectionnées aux points isobestiques de réduction des taux d'hémoglobine et d'oxyhémoglobine.

**12.** Système selon la revendication 10 ou 11, dans lequel lesdites première et deuxième caractéristiques d'extinction sont constituées par des première et deuxième courbes.

**13.** Système selon la revendication 10 ou 11, dans lequel lesdites première et deuxième caractéristiques d'extinction sont constituées par des premier et deuxième décalages.

**14.** Système selon la revendication 10 ou 11, dans lequel lesdites première et deuxième caractéristiques d'extinction sont constituées par des première et deuxième caractéristiques de linéarité.

**15.** Système selon la revendication 10 ou 11, dans lequel ledit premier signe est différent dudit deuxième signe.

**16.** Système selon l'une quelconque des revendications 10 à 15, dans lequel lesdits premiers et deuxièmes moyens de photodétection sont formés par un unique photodétecteur.

**17.** Système selon l'une quelconque des revendications 10 à 16, le système, en outre, comprenant :

- un troisième émetteur positionné sur lesdits moyens de réception de conduit pour émettre une troisième longueur d'onde de rayonnement qui fera l'objet d'une extinction par un constituant biologique en concurrence dans le sang, d'une manière caractéristique dudit constituant biologique en concurrence, et ayant une caractéristique d'extinction, relativement aux constituants autres que ledit constituant biologique en concurrence, sensiblement différente d'une desdites première et deuxième caractéristiques d'extinction ;
- des deuxièmes moyens directeurs pour diriger un rayonnement ayant la troisième longueur d'onde jusque dans le conduit de sang :

  - des moyens de détection pour détecter l'intensité du rayonnement, ayant la troisième longueur d'onde, faisant l'objet d'une extinction par le sang dans le conduit de sang ;
  - des moyens pour effectuer des opérations sur les intensités des rayonnements détectés, ayant les première, deuxième et troisième longueurs d'ondes, de sorte que les variations spatiales, géométriques et relatives aux tissus soient éliminées pour chaque longueur d'onde de rayonnement ; et
  - des moyens pour compenser l'effet du deuxième constituant biologique en effectuant des opérations sur les intensités des rayonnements détectés ayant les première, deuxième et troisième longueurs d'ondes.

**18.** Système selon l'une quelconque des revendications 10 à 17, dans lequel lesdits moyens de comparaison sont configurés pour manipuler mathématiquement les intensités détectées des première et deuxième longueurs d'ondes de rayonnements avec une fonction polynomiale afin de déterminer la valeur du taux d'hématocrite.

**19.** Système selon l'une quelconque des revendications 10 à 18, dans lequel lesdits moyens de comparaison comprennent des moyens pour établir le rapport des taux $\Delta I / I$ pour chacune des première et deuxième longueurs d'ondes de rayonnements, $\Delta I$ représentant une dérivée dans le temps d'une composante d'intensité transmise de manière pulsatoire et I représentant une intensité transmise en régime établi.

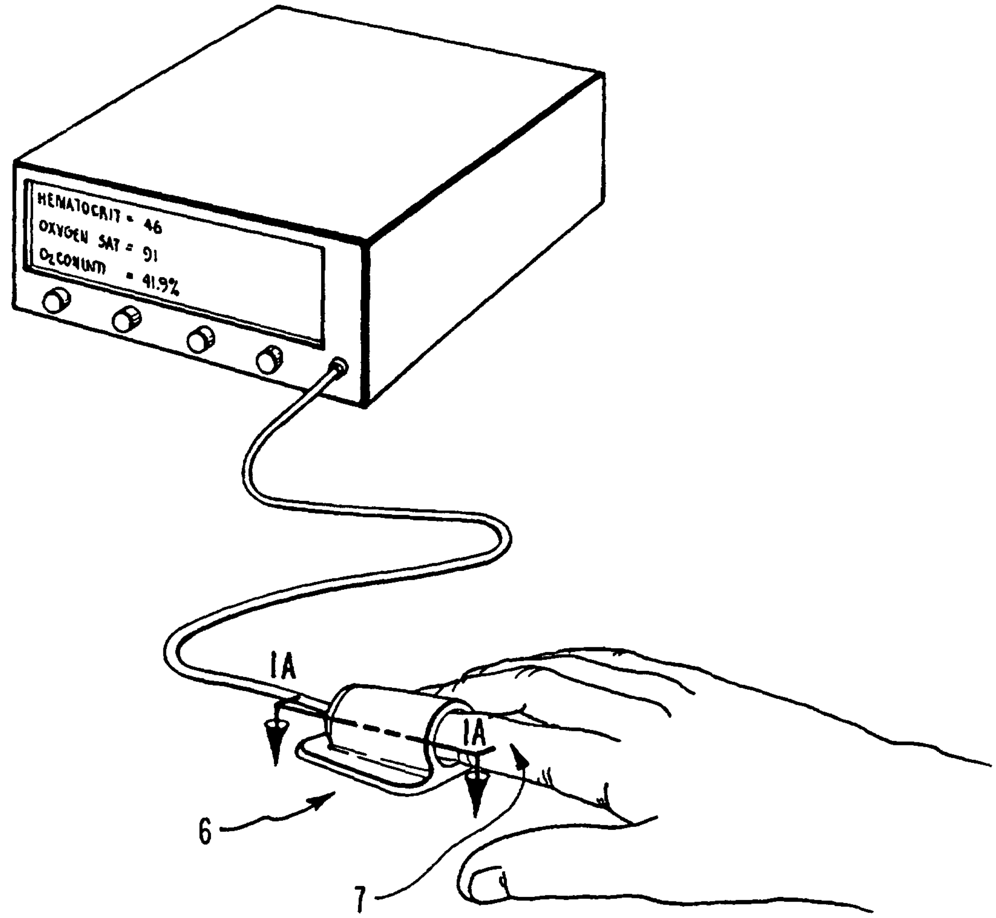
HEMATOCRIT = 46
OXYGEN SAT = 91
O2 CONTENT = 41.9%
1A
1A
6
7

FIG. 1

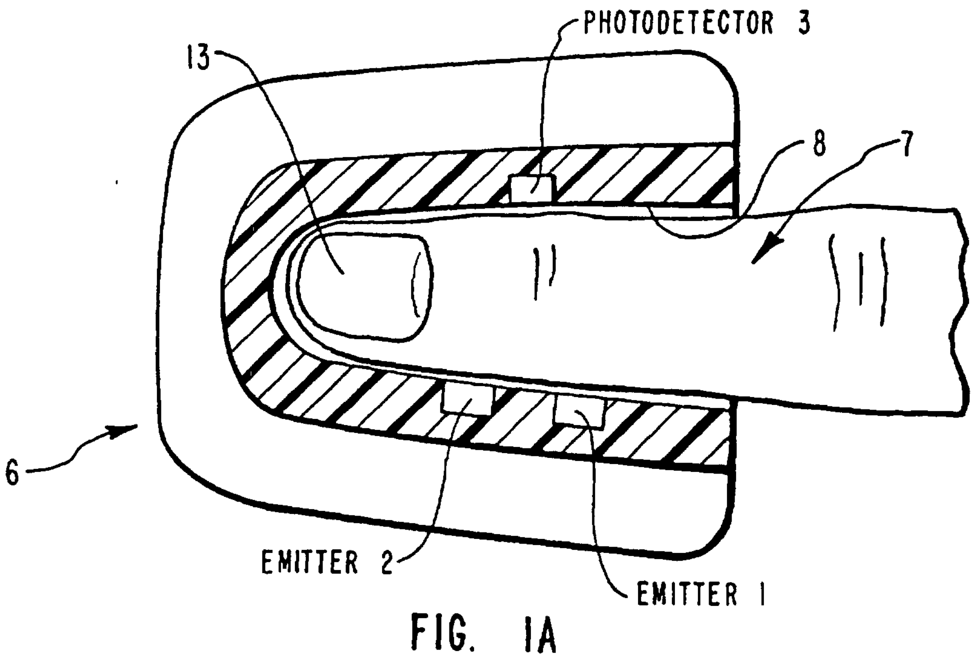
PHOTODETECTOR 3
13
8
7
6
EMITTER 2
EMITTER 1

FIG. 1A

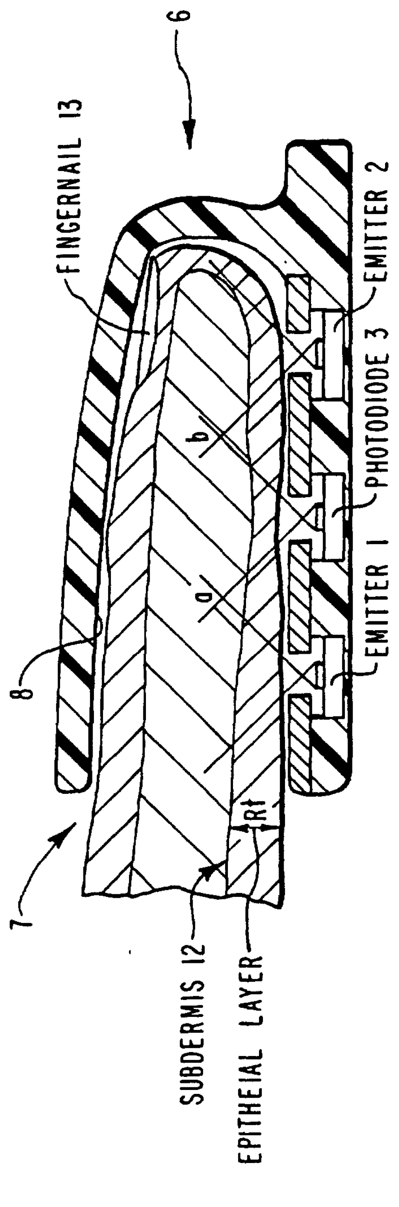
6
7
8
FINGERNAIL 13
SUBDERMIS 12
EPITHEIAL LAYER
R
a
b
EMITTER 1
PHOTODIODE 3
EMITTER 2

FIG. 1B

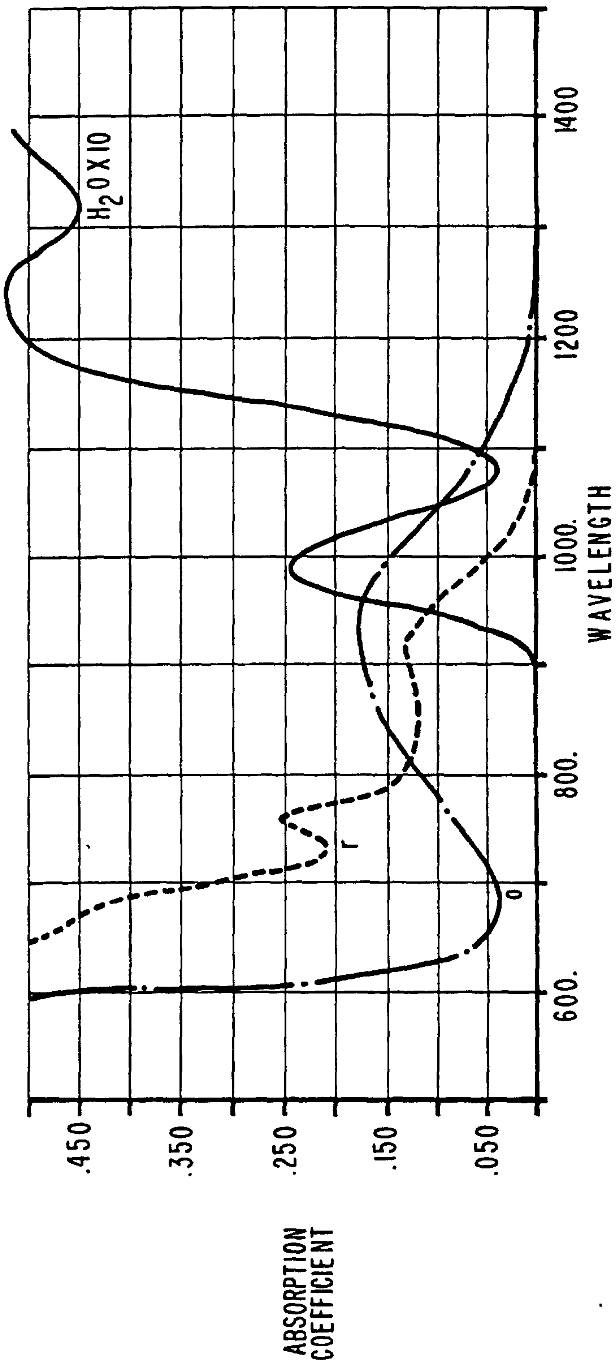
ABSORPTION COEFFICIENT
.450
.350
.250
.150
.050
600.
800.
1000.
1200
1400
WAVELENGTH
r
o
$H_2O \times 10$

FIG. 2

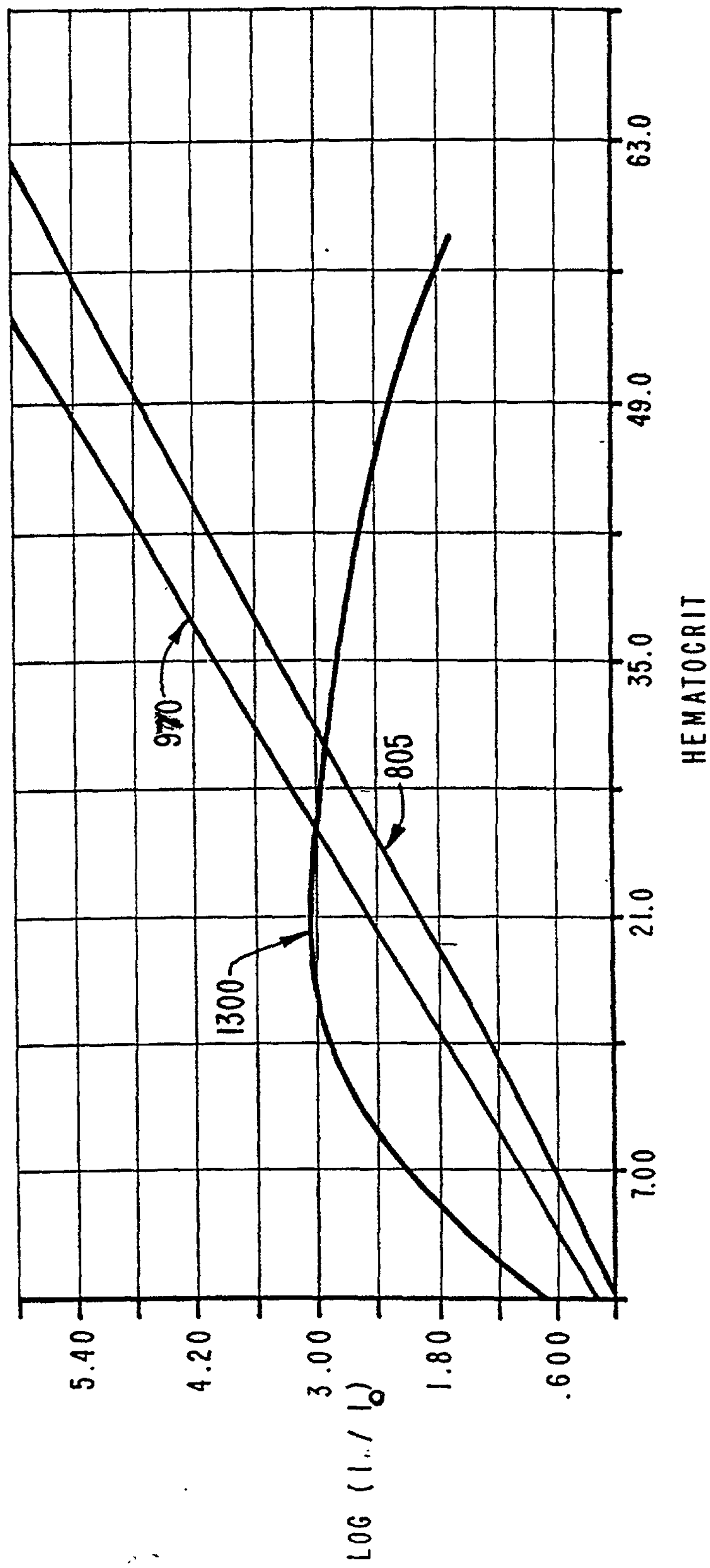
5.40
4.20
3.00
1.80
.600
LOG (I / I0)
7.00
21.0
35.0
49.0
63.0
HEMATOCRIT
970
805
1300

FIG. 3

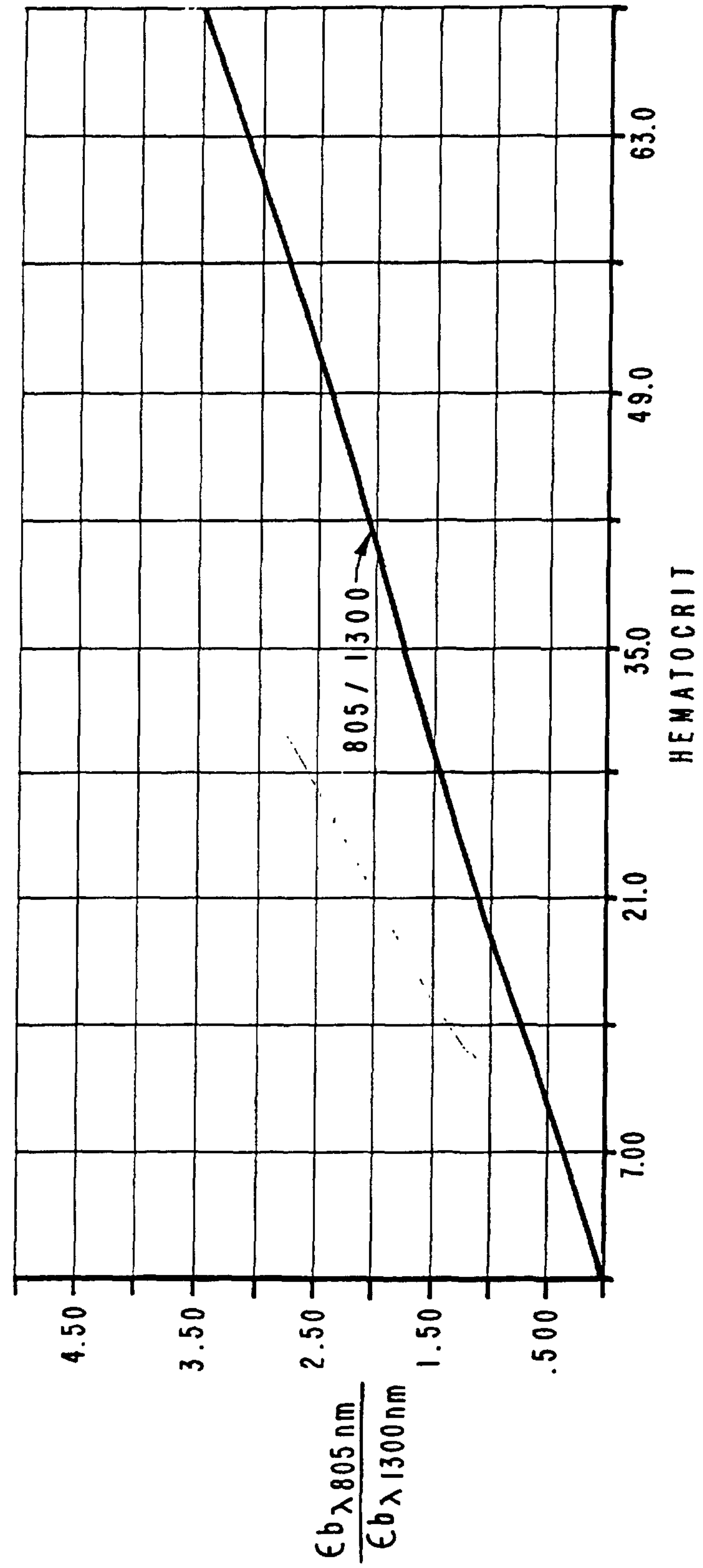
4.50
3.50
2.50
1.50
.500
€bλ805 nm / €bλ1300nm
7.00
21.0
35.0
49.0
63.0
HEMATOCRIT
805/1300

FIG. 4

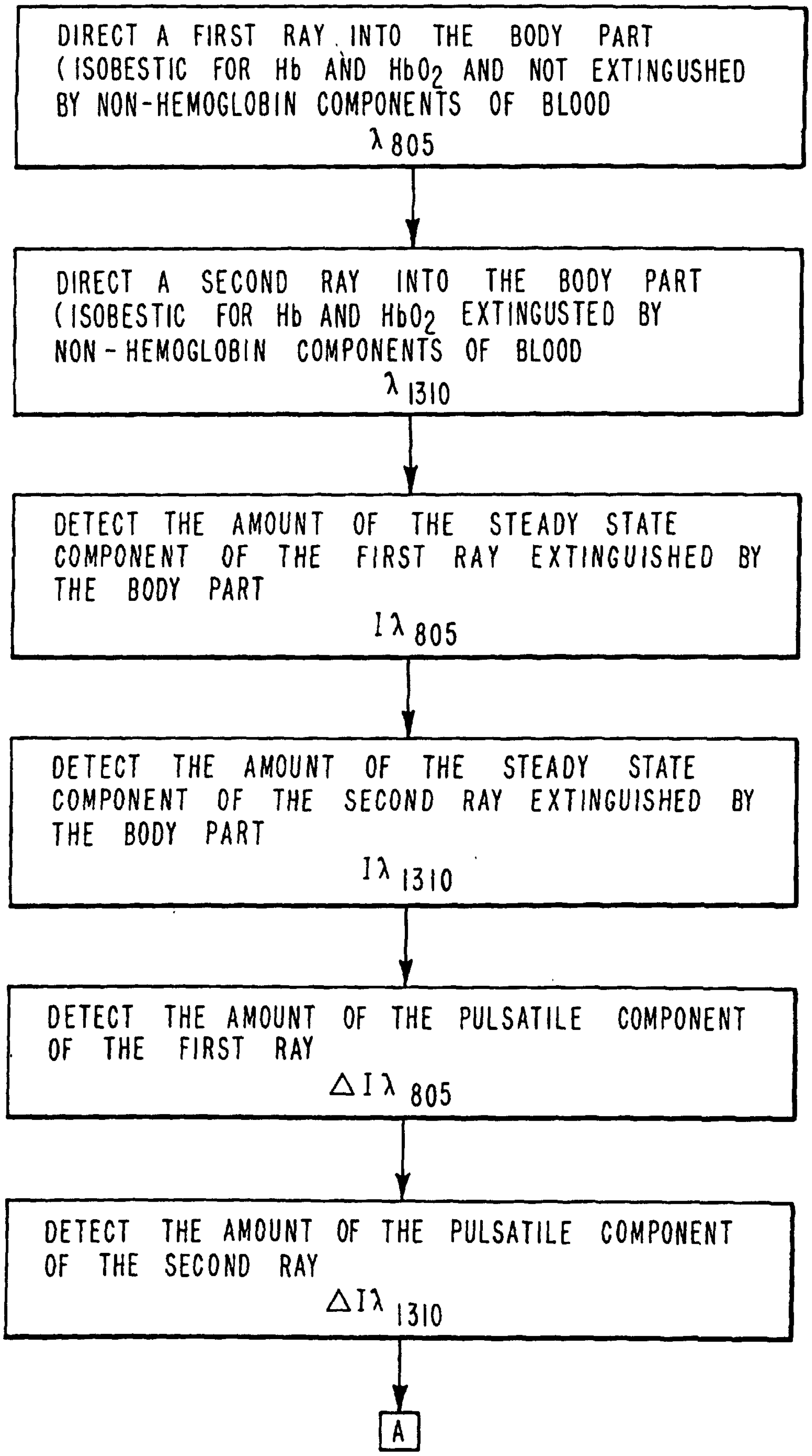
DIRECT A FIRST RAY INTO THE BODY PART (ISOBESTIC FOR Hb AND $HbO_2$ AND NOT EXTINGUSHED BY NON-HEMOGLOBIN COMPONENTS OF BLOOD
$\lambda_{805}$
DIRECT A SECOND RAY INTO THE BODY PART (ISOBESTIC FOR Hb AND $HbO_2$ EXTINGUSTED BY NON-HEMOGLOBIN COMPONENTS OF BLOOD
$\lambda_{1310}$
DETECT THE AMOUNT OF THE STEADY STATE COMPONENT OF THE FIRST RAY EXTINGUISHED BY THE BODY PART
$I\lambda_{805}$
DETECT THE AMOUNT OF THE STEADY STATE COMPONENT OF THE SECOND RAY EXTINGUISHED BY THE BODY PART
$I\lambda_{1310}$
DETECT THE AMOUNT OF THE PULSATILE COMPONENT OF THE FIRST RAY
$\Delta I\lambda_{805}$
DETECT THE AMOUNT OF THE PULSATILE COMPONENT OF THE SECOND RAY
$\Delta I\lambda_{1310}$
A

FIG. 5A

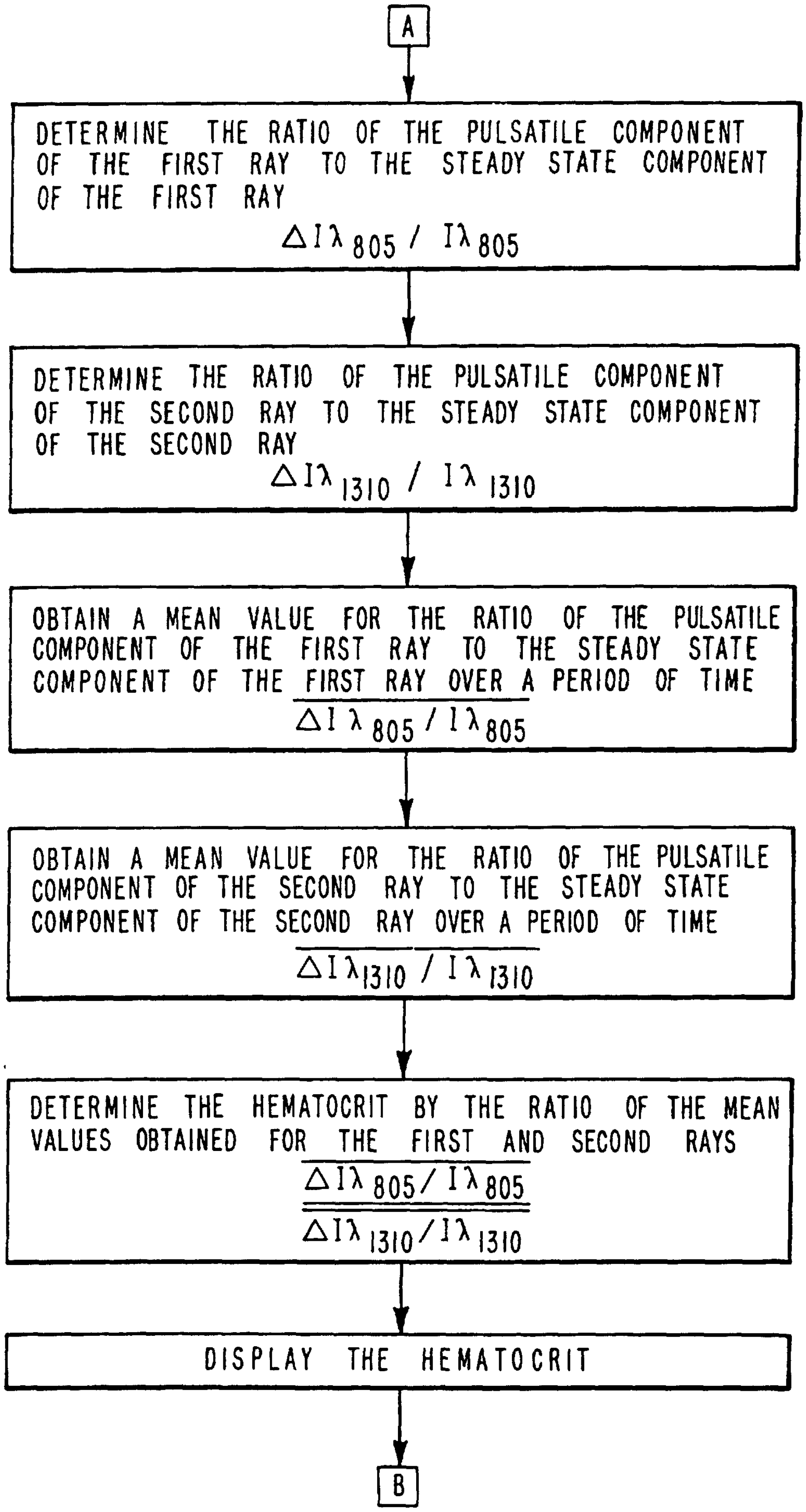
A
DETERMINE THE RATIO OF THE PULSATILE COMPONENT OF THE FIRST RAY TO THE STEADY STATE COMPONENT OF THE FIRST RAY
$\Delta I\lambda_{805} / I\lambda_{805}$
DETERMINE THE RATIO OF THE PULSATILE COMPONENT OF THE SECOND RAY TO THE STEADY STATE COMPONENT OF THE SECOND RAY
$\Delta I\lambda_{1310} / I\lambda_{1310}$
OBTAIN A MEAN VALUE FOR THE RATIO OF THE PULSATILE COMPONENT OF THE FIRST RAY TO THE STEADY STATE COMPONENT OF THE FIRST RAY OVER A PERIOD OF TIME
$\overline{\Delta I\lambda_{805} / I\lambda_{805}}$
OBTAIN A MEAN VALUE FOR THE RATIO OF THE PULSATILE COMPONENT OF THE SECOND RAY TO THE STEADY STATE COMPONENT OF THE SECOND RAY OVER A PERIOD OF TIME
$\overline{\Delta I\lambda_{1310} / I\lambda_{1310}}$
DETERMINE THE HEMATOCRIT BY THE RATIO OF THE MEAN VALUES OBTAINED FOR THE FIRST AND SECOND RAYS
$\frac{\overline{\Delta I\lambda_{805} / I\lambda_{805}}}{\overline{\Delta I\lambda_{1310} / I\lambda_{1310}}}$
DISPLAY THE HEMATOCRIT
B

FIG. 5B

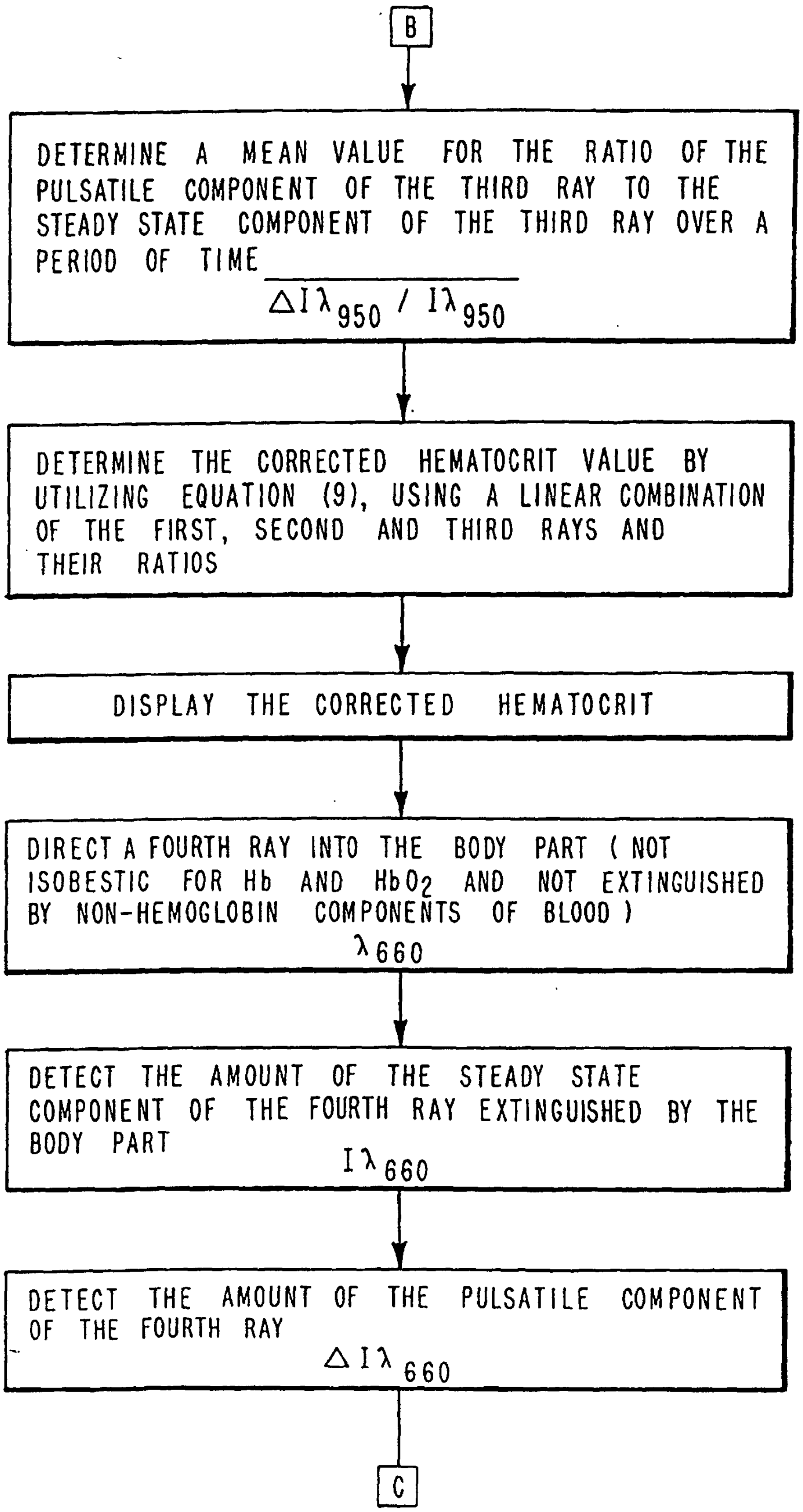
B
DETERMINE A MEAN VALUE FOR THE RATIO OF THE PULSATILE COMPONENT OF THE THIRD RAY TO THE STEADY STATE COMPONENT OF THE THIRD RAY OVER A PERIOD OF TIME
$\Delta I\lambda_{950} / I\lambda_{950}$
DETERMINE THE CORRECTED HEMATOCRIT VALUE BY UTILIZING EQUATION (9), USING A LINEAR COMBINATION OF THE FIRST, SECOND AND THIRD RAYS AND THEIR RATIOS
DISPLAY THE CORRECTED HEMATOCRIT
DIRECT A FOURTH RAY INTO THE BODY PART ( NOT ISOBESTIC FOR Hb AND $HbO_2$ AND NOT EXTINGUISHED BY NON-HEMOGLOBIN COMPONENTS OF BLOOD )
$\lambda_{660}$
DETECT THE AMOUNT OF THE STEADY STATE COMPONENT OF THE FOURTH RAY EXTINGUISHED BY THE BODY PART
$I\lambda_{660}$
DETECT THE AMOUNT OF THE PULSATILE COMPONENT OF THE FOURTH RAY
$\Delta I\lambda_{660}$
C

FIG. 5C

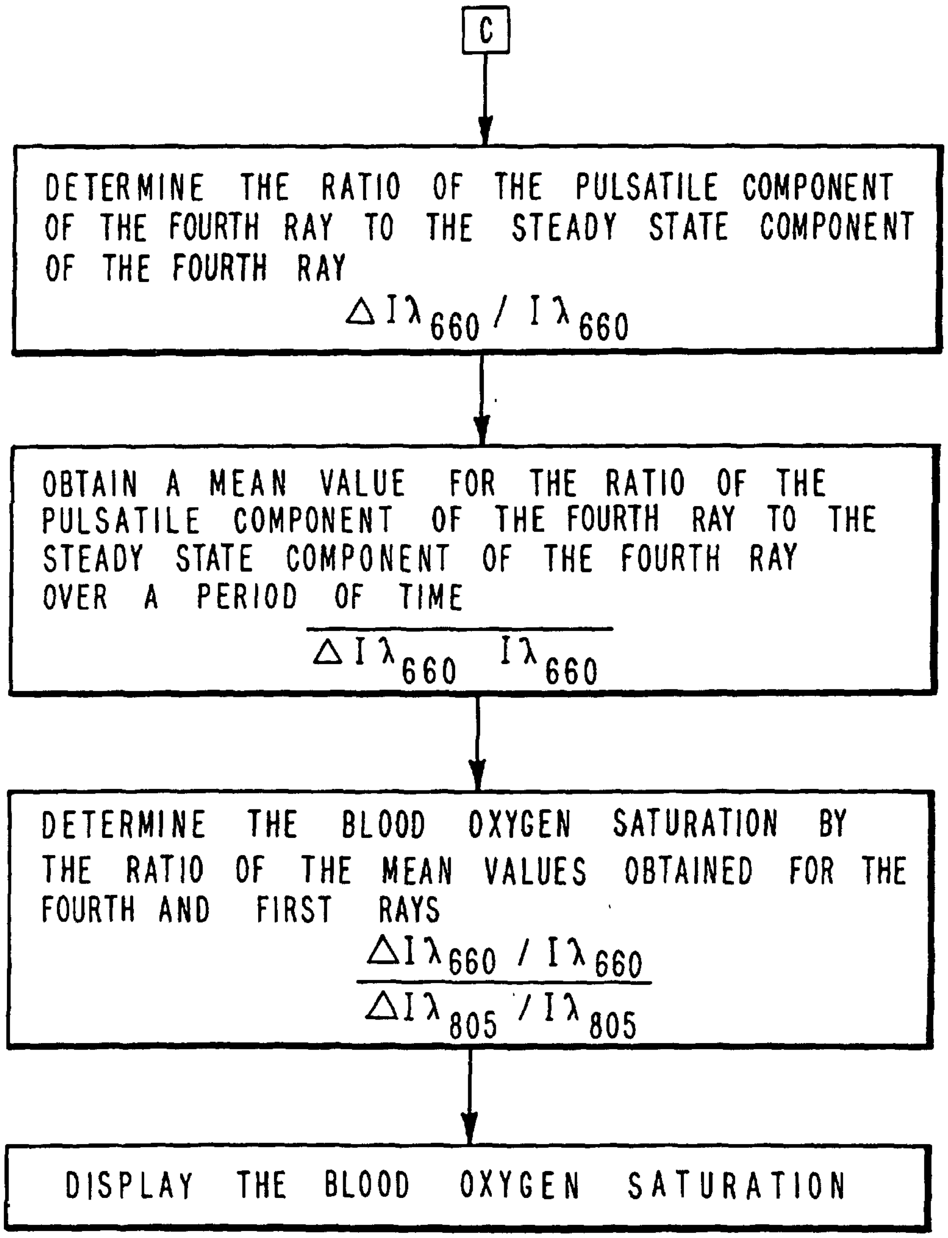
C
DETERMINE THE RATIO OF THE PULSATILE COMPONENT OF THE FOURTH RAY TO THE STEADY STATE COMPONENT OF THE FOURTH RAY
$\Delta I\lambda_{660} / I\lambda_{660}$
OBTAIN A MEAN VALUE FOR THE RATIO OF THE PULSATILE COMPONENT OF THE FOURTH RAY TO THE STEADY STATE COMPONENT OF THE FOURTH RAY OVER A PERIOD OF TIME
$\overline{\Delta I\lambda_{660} \quad I\lambda_{660}}$
DETERMINE THE BLOOD OXYGEN SATURATION BY THE RATIO OF THE MEAN VALUES OBTAINED FOR THE FOURTH AND FIRST RAYS
$\frac{\Delta I\lambda_{660} / I\lambda_{660}}{\Delta I\lambda_{805} / I\lambda_{805}}$
DISPLAY THE BLOOD OXYGEN SATURATION

FIG. 5D

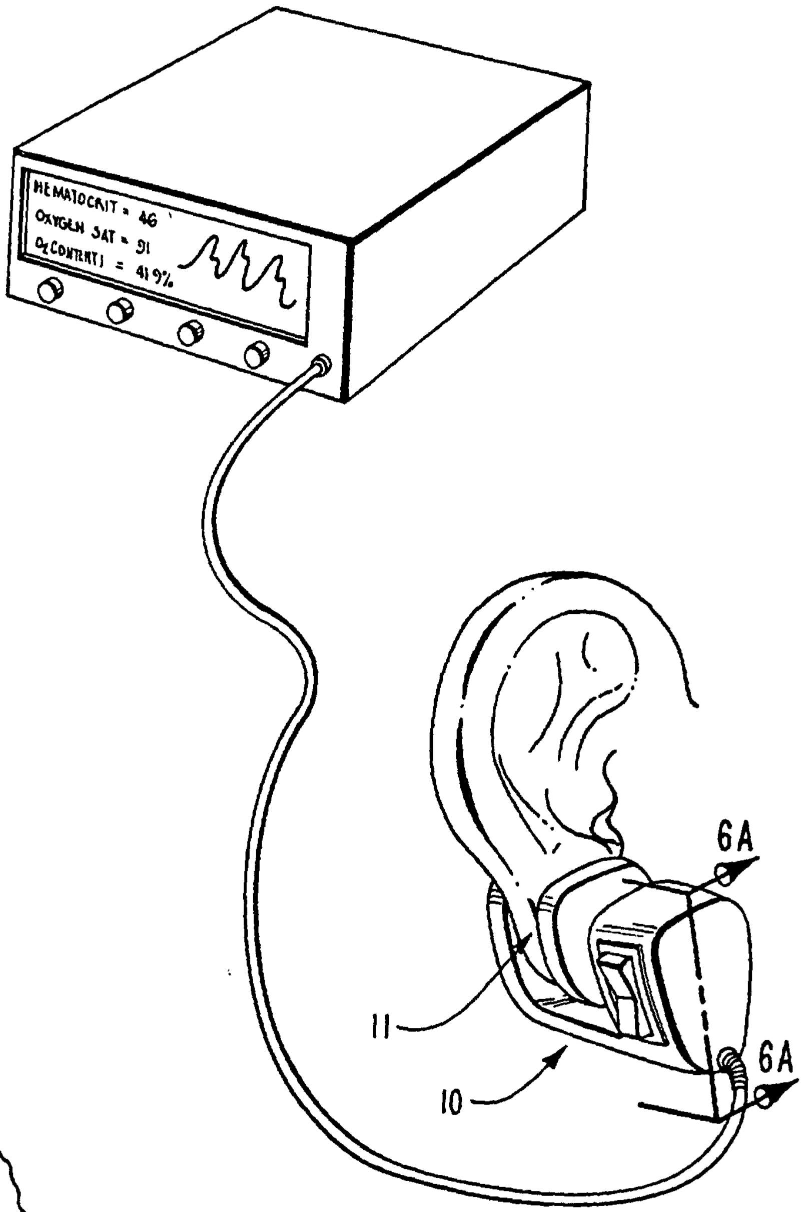
HEMATOCRIT = 46
OXYGEN SAT = 91
O2 CONTENT = 41.9%
6A
6A
11
10

FIG. 6

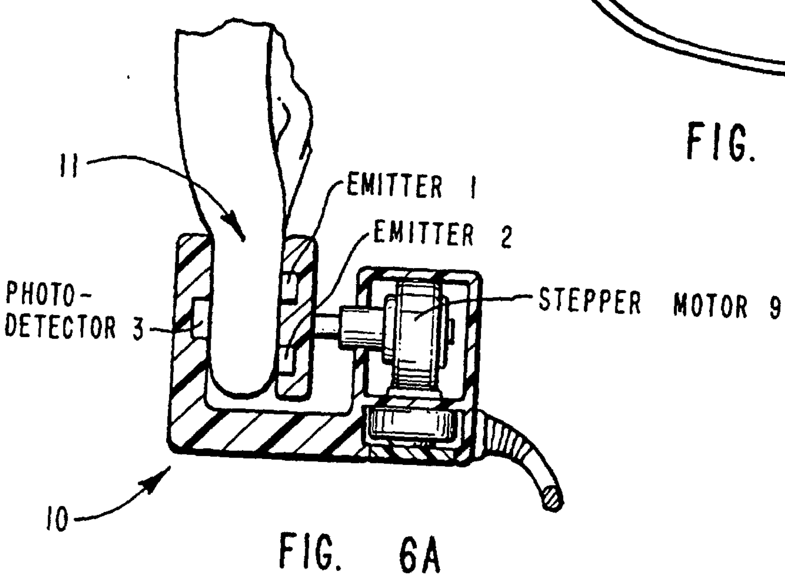
11
EMITTER 1
EMITTER 2
PHOTO-
DETECTOR 3
STEPPER MOTOR 9
10

FIG. 6A

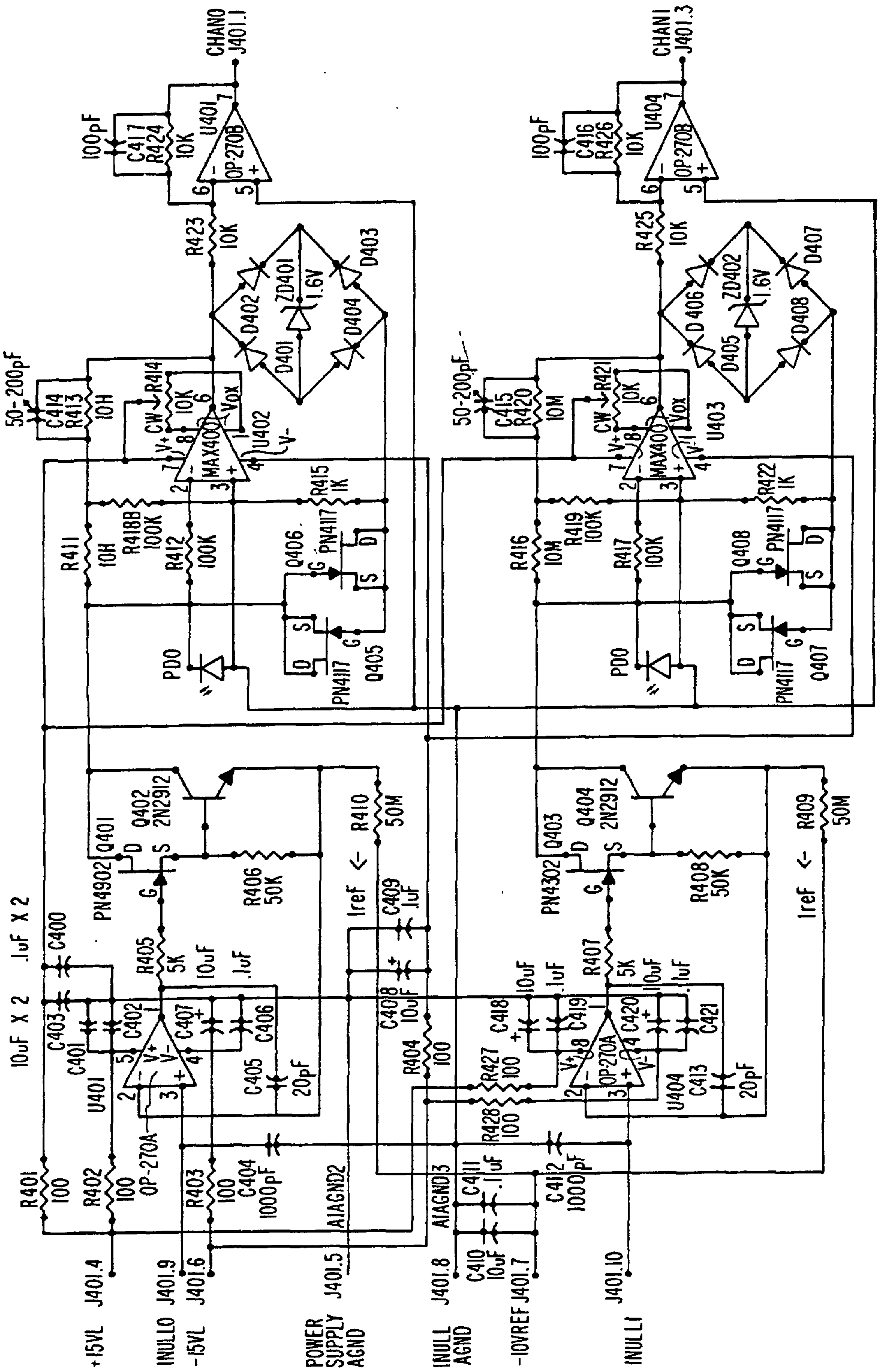

+15VL J401.4
INULLO J401.9
-15VL J401.6
POWER SUPPLY AGND J401.5
INULL AGND J401.8
-10VREF J401.7
INULL1 J401.10
CHAN0 J401.1
CHAN1 J401.3
10uF X 2
.1uF X 2
R401 100
R402 100
R403 100
R404 100
R405 5K
R406 50K
R407 5K
R408 50K
R409 50M
R410 50M
R411 10M
R412 100K
R413 10M
R414 10K
R415 1K
R416 10M
R417 100K
R418B 100K
R419 100K
R420 10M
R421 10K
R422 1K
R423 10K
R424 10K
R425 10K
R426 10K
R427 100
R428 100
C404 1000pF
C405 20pF
C408 10uF
C409 .1uF
C410 10uF
C411 .1uF
C412 1000pF
C413 20pF
C414 50-200pF
C415 50-200pF
C416 100pF
C417 100pF
U401 OP-270A
U401 OP-270B
U402 MAX400
U403 MAX400
U404 OP-270A
U404 OP-270B
Q401 PN4902
Q402 2N2912
Q403 PN4302
Q404 2N2912
Q405 PN4117
Q406 PN4117
Q407 PN4117
Q408 PN4117
D401 D402 D403 D404
ZD401 1.6V
D405 D406 D407 D408
ZD402 1.6V
PD0
IreF
AIAGND2
AIAGND3
CW
Vox


FIG. 7

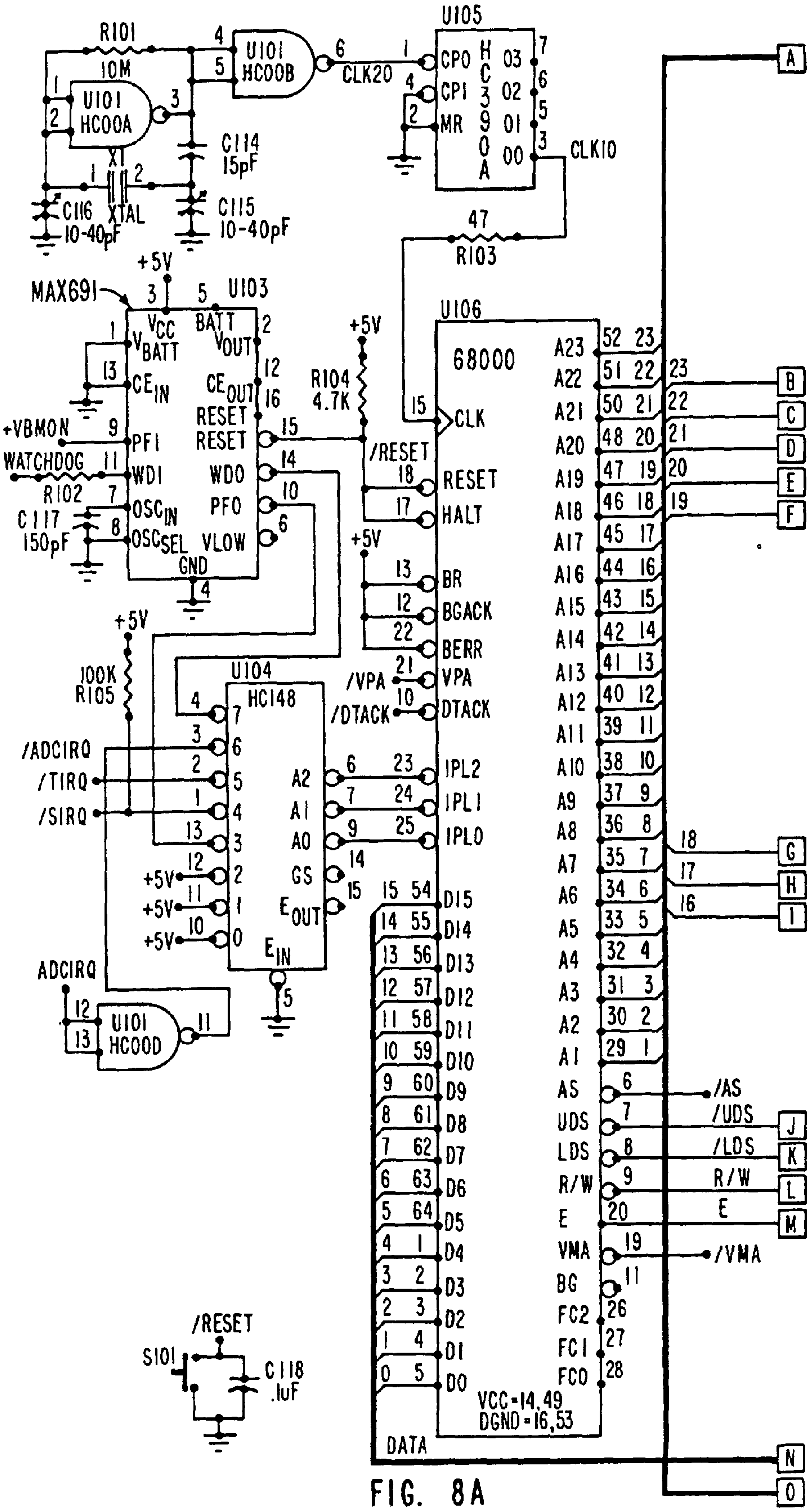
R101
10M
U101
HC00A
U101
HC00B
CLK20
C114
15pF
X1
XTAL
C116
10-40pF
C115
10-40pF
U105
CP0
CP1
MR
HC390A
03
02
01
00
CLK10
47
R103
+5V
MAX691
U103
VCC
BATT
VBATT
VOUT
CEIN
CEOUT
RESET
+VBMON
PFI
WATCHDOG
WDI
WDO
R102
OSCIN
PFO
C117
150pF
OSCSEL
VLOW
GND
R104
4.7K
/RESET
U106
68000
CLK
RESET
HALT
BR
BGACK
BERR
/VPA
VPA
/DTACK
DTACK
IPL2
IPL1
IPL0
100K
R105
U104
HC148
/ADCIRQ
/TIRQ
/SIRQ
A2
A1
A0
GS
EOUT
EIN
ADCIRQ
U101
HC00D
D15
D14
D13
D12
D11
D10
D9
D8
D7
D6
D5
D4
D3
D2
D1
D0
A23
A22
A21
A20
A19
A18
A17
A16
A15
A14
A13
A12
A11
A10
A9
A8
A7
A6
A5
A4
A3
A2
A1
AS
UDS
LDS
R/W
E
VMA
BG
FC2
FC1
FC0
/AS
/UDS
/LDS
R/W
E
/VMA
VCC=14,49
DGND=16,53
DATA
S101
/RESET
C118
.1uF
A
B
C
D
E
F
G
H
I
J
K
L
M
N
O

FIG. 8A

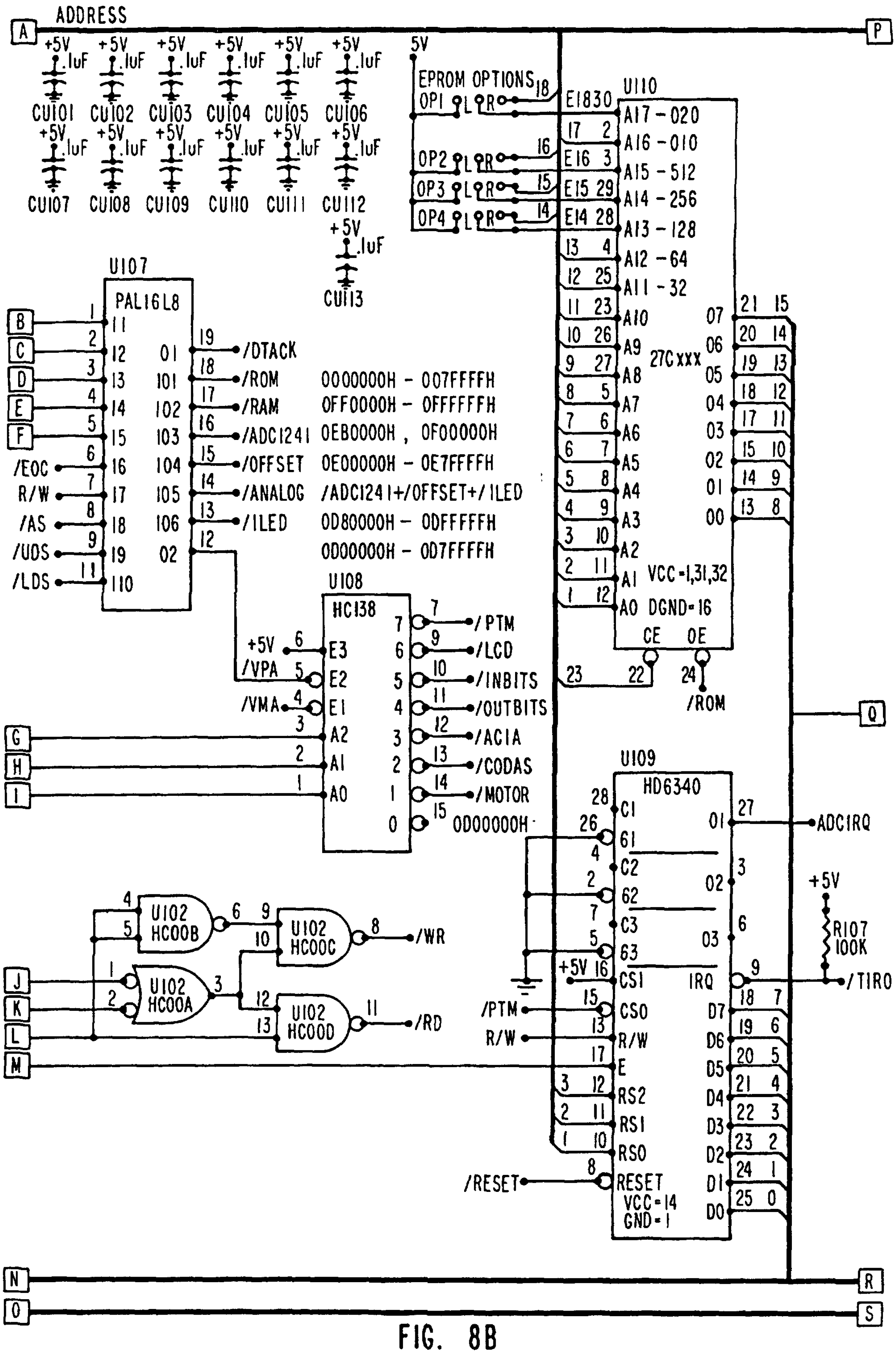

ADDRESS
EPROM OPTIONS
U110
27CXXX
VCC=1,31,32
DGND=16
U107
PAL16L8
/DTACK
/ROM 0000000H - 007FFFFH
/RAM 0FF0000H - 0FFFFFFH
/ADC1241 0EB0000H , 0F00000H
/OFFSET 0E00000H - 0E7FFFFH
/ANALOG /ADC1241+/OFFSET+/ILED
/ILED 0D80000H - 0DFFFFFH
0D00000H - 0D7FFFFH
U108
HC138
/PTM
/LCD
/INBITS
/OUTBITS
/ACIA
/CODAS
/MOTOR
0000000H
U109
HD6340
ADC1RQ
R107 100K
/TIRQ
/RESET
VCC=14
GND=1
U102 HC00A
U102 HC00B
U102 HC00C
U102 HC00D
/WR
/RD


FIG. 8B

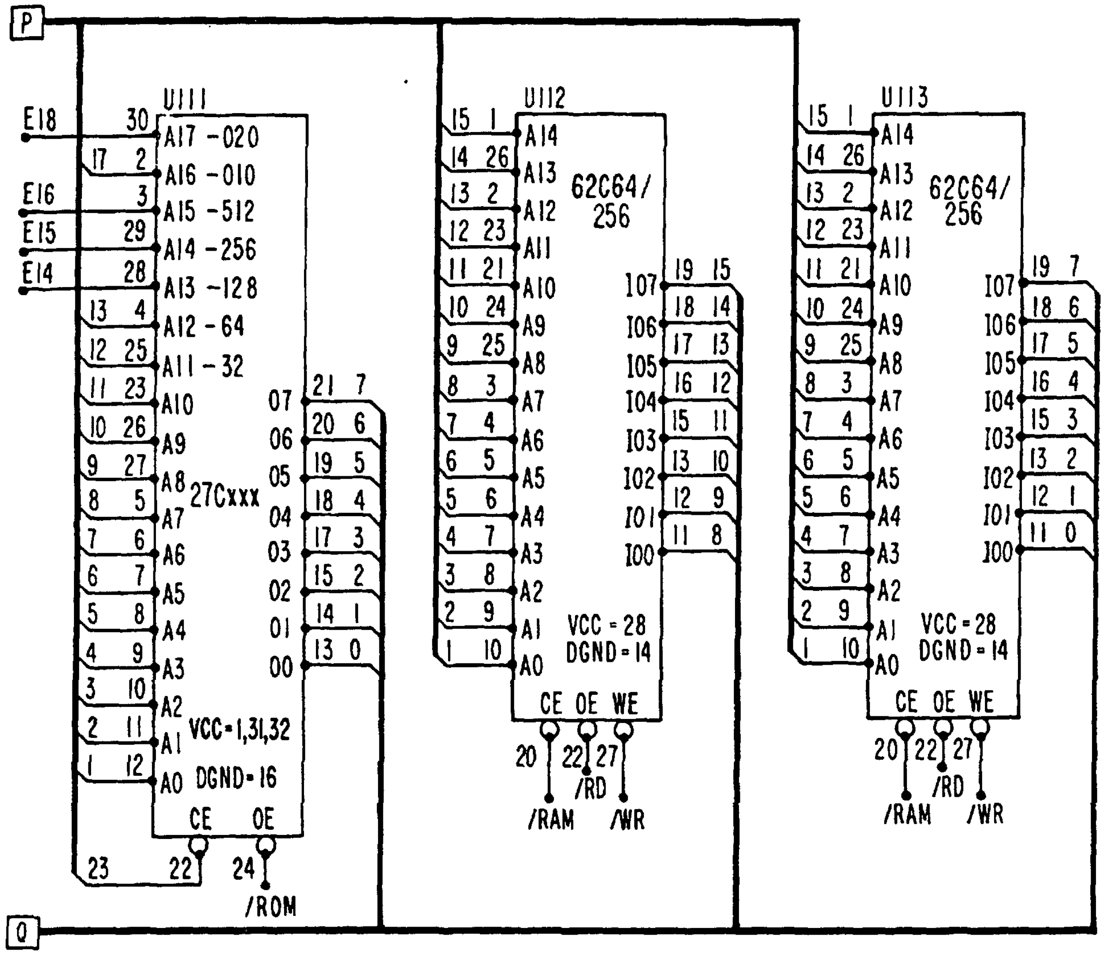


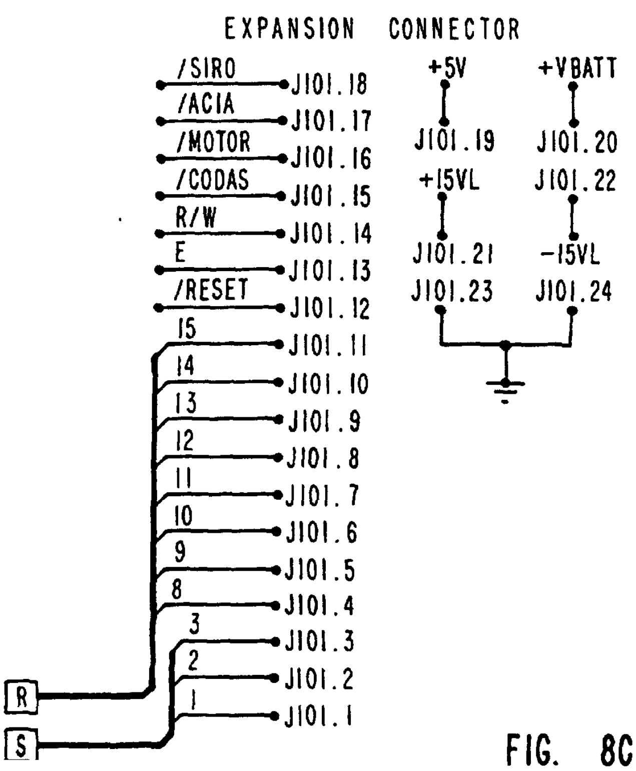


| EPROM SIZE | OP1 | OP2 | OP3 | OP4 |
|---|---|---|---|---|
| 27C32 | X | L | X | X |
| 27C64 | L | X | L | L |
| 27C128 | L | R | L | L |
| 27C256 | L | R | L | R |
| 27C512 | L | R | R | R |
| 27C010 | X | R | R | R |
| 27C020 | R | R | R | R |

FIG. 8C

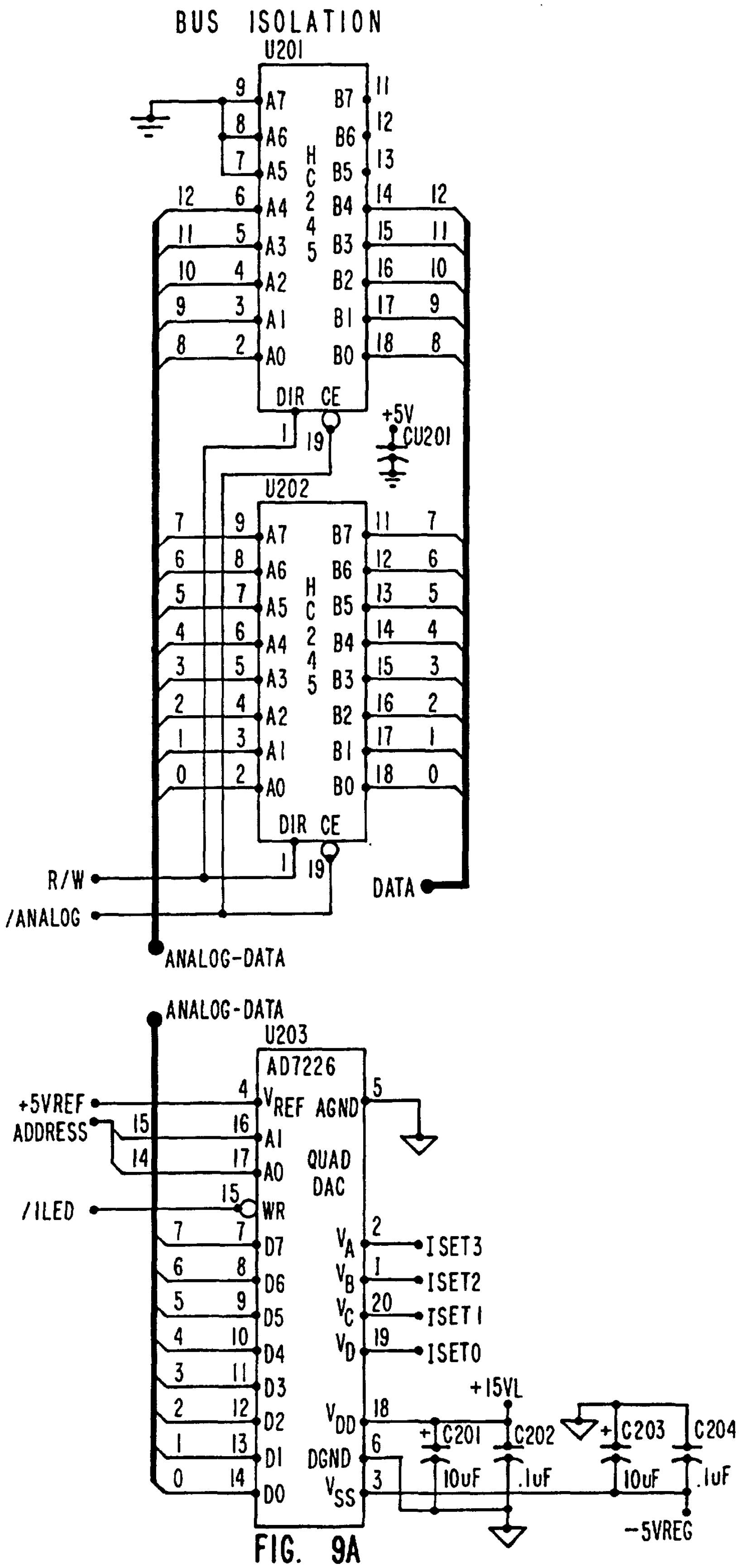
BUS ISOLATION
U201
HC245
+5V
CU201
U202
HC245
R/W
/ANALOG
ANALOG-DATA
DATA
ANALOG-DATA
U203
AD7226
QUAD DAC
+5VREF
ADDRESS
/ILED
ISET3
ISET2
ISET1
ISET0
+15VL
C201
10uF
C202
.1uF
C203
10uF
C204
.1uF
-5VREG

FIG. 9A

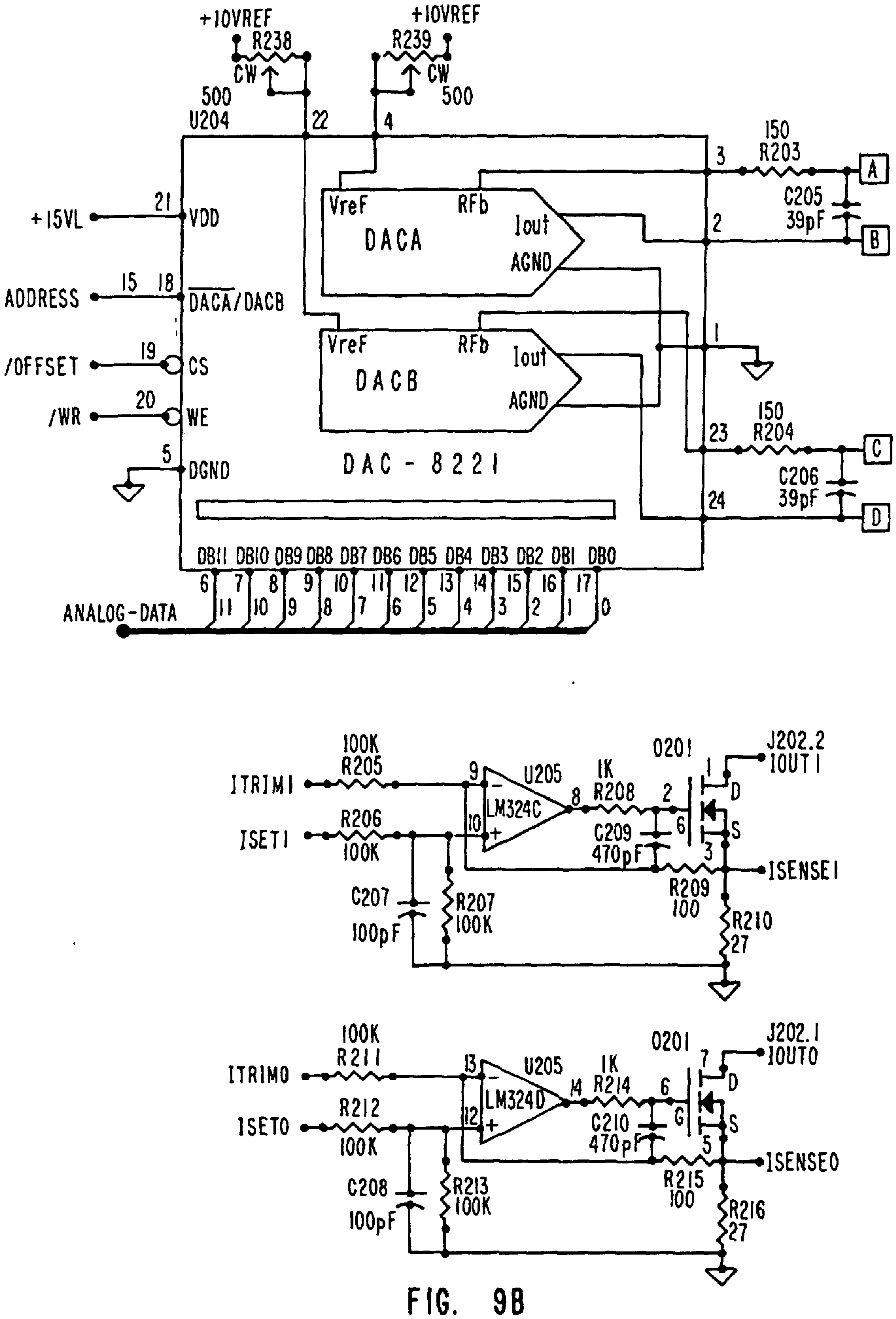

+10VREF
R238
CW
500
U204
22
4
+10VREF
R239
CW
500
150
R203
3
A
C205
39pF
2
B
+15VL
21
VDD
VreF
RFb
DACA
Iout
AGND
ADDRESS
15
18
DACA/DACB
VreF
RFb
DACB
Iout
AGND
1
/OFFSET
19
CS
/WR
20
WE
5
DGND
DAC - 8221
150
R204
23
C
C206
39pF
24
D
DB11 DB10 DB9 DB8 DB7 DB6 DB5 DB4 DB3 DB2 DB1 DB0
6 7 8 9 10 11 12 13 14 15 16 17
11 10 9 8 7 6 5 4 3 2 1 0
ANALOG-DATA
100K
R205
ITRIM1
9
U205
LM324C
8
1K
R208
2
Q201
1
J202.2
IOUT1
D
S
6
ISET1
R206
100K
10
C209
470pF
3
ISENSE1
C207
100pF
R207
100K
R209
100
R210
27
100K
R211
ITRIM0
13
U205
LM324D
14
1K
R214
6
Q201
7
J202.1
IOUT0
D
G
S
ISET0
R212
100K
12
C210
470pF
5
ISENSE0
C208
100pF
R213
100K
R215
100
R216
27


FIG. 9B

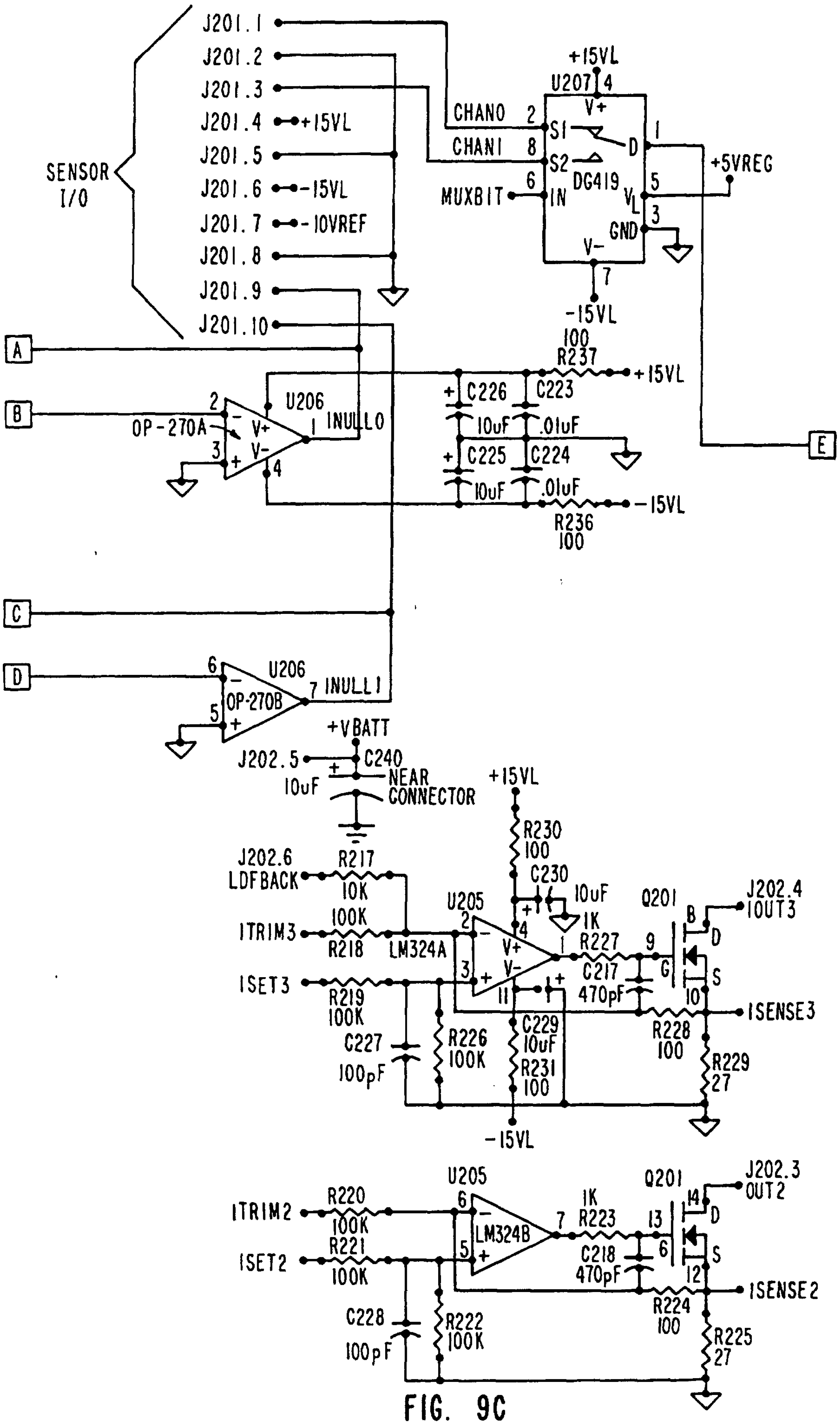
J201.1
J201.2
J201.3
J201.4 +15VL
J201.5
J201.6 -15VL
J201.7 -10VREF
J201.8
J201.9
J201.10
SENSOR I/O
+15VL
U207
CHAN0
CHAN1
MUXBIT
DG419
+5VREG
-15VL
A
B
C
D
E
U206
OP-270A
INULL0
OP-270B
INULL1
R237 100
R236 100
C226 10uF
C223 .01uF
C225 10uF
C224 .01uF
+VBATT
J202.5
C240 10uF
NEAR CONNECTOR
J202.6 LDFBACK
R217 10K
ITRIM3
R218 100K
ISET3
R219 100K
U205
LM324A
R230 100
C230 10uF
Q201
J202.4 IOUT3
1K R227
C217 470pF
ISENSE3
R228 100
R229 27
C227 100pF
R226 100K
C229 10uF
R231 100
ITRIM2
R220 100K
ISET2
R221 100K
LM324B
1K R223
C218 470pF
J202.3 OUT2
ISENSE2
R224 100
R225 27
C228 100pF
R222 100K

FIG. 9C

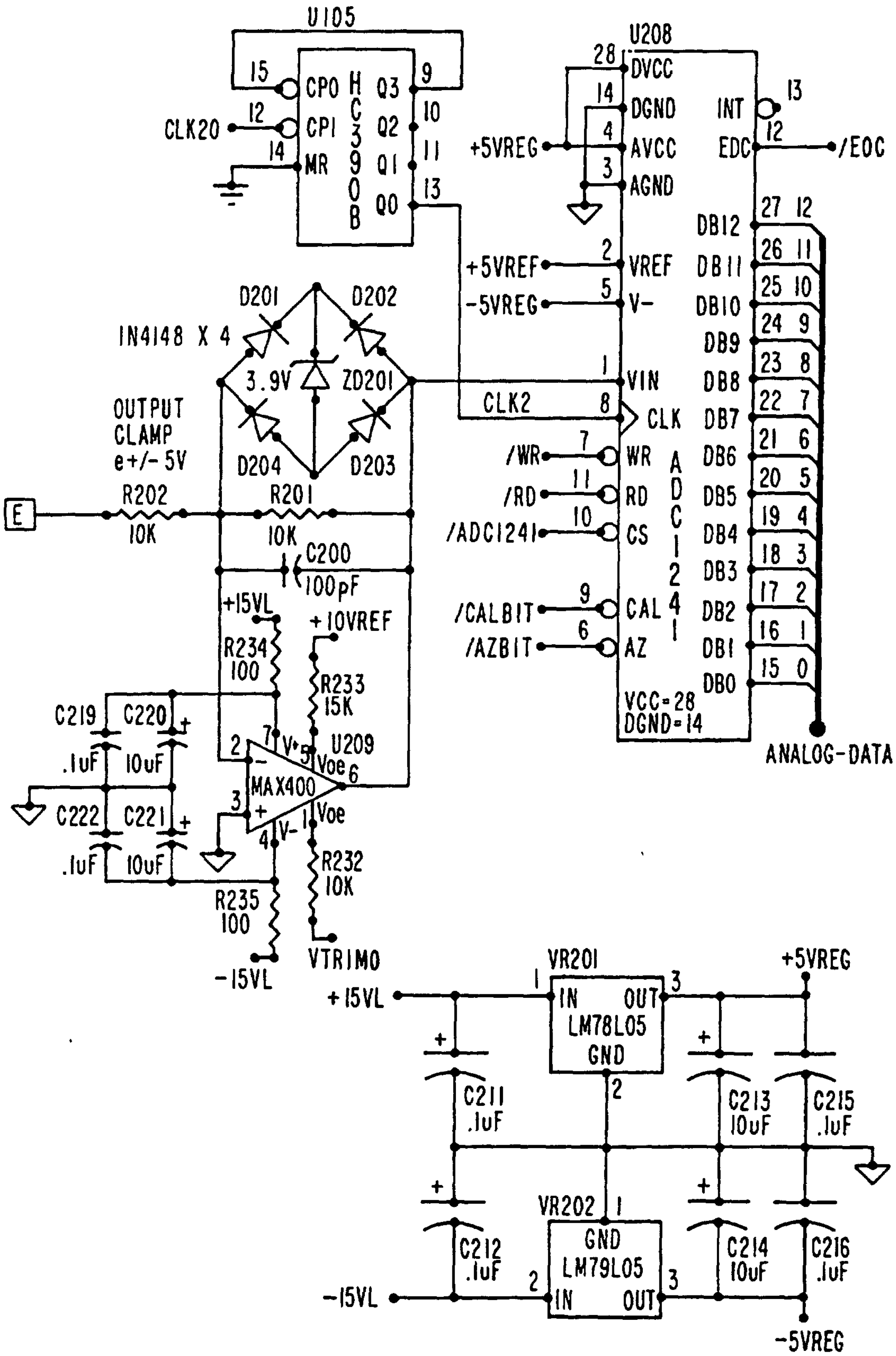
U105
CP0
CP1
MR
HC390B
Q3
Q2
Q1
Q0
CLK20
U208
DVCC
DGND
AVCC
AGND
+5VREG
INT
EDC
/EOC
+5VREF
-5VREG
VREF
V-
VIN
CLK
CLK2
/WR
/RD
/ADC1241
/CALBIT
/AZBIT
WR
RD
CS
CAL
AZ
ADC1241
DB12
DB11
DB10
DB9
DB8
DB7
DB6
DB5
DB4
DB3
DB2
DB1
DB0
VCC=28
DGND=14
ANALOG-DATA
D201
D202
D203
D204
IN4148 X 4
3.9V
ZD201
OUTPUT
CLAMP
e+/-5V
E
R202
10K
R201
10K
C200
100pF
+15VL
+10VREF
R234
100
R233
15K
C219
C220
.1uF
10uF
U209
MAX400
Voe
V+
V-
C222
C221
.1uF
10uF
R235
100
R232
10K
-15VL
VTRIMO
VR201
IN
OUT
LM78L05
GND
+15VL
+5VREG
C211
.1uF
C213
10uF
C215
.1uF
VR202
LM79L05
C212
.1uF
C214
10uF
C216
.1uF
-15VL
-5VREG

FIG. 9D

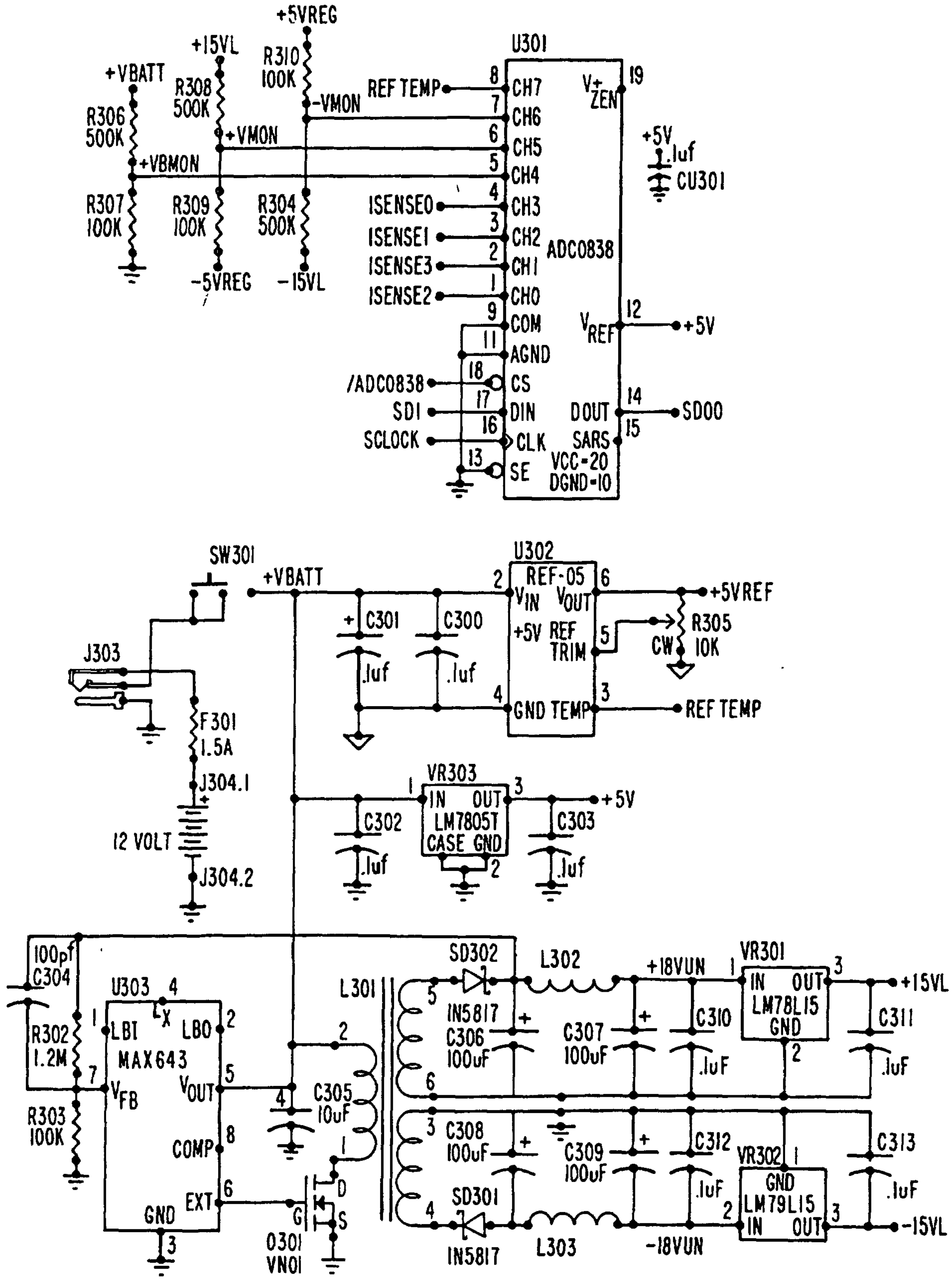

+5VREG
+15VL
+VBATT
R310
100K
R308
500K
R306
500K
-VMON
+VMON
+VBMON
R307
100K
R309
100K
R304
500K
-5VREG
-15VL
U301
REF TEMP
ISENSE0
ISENSE1
ISENSE3
ISENSE2
CH7
CH6
CH5
CH4
CH3
CH2
CH1
CH0
COM
AGND
CS
DIN
CLK
SE
ADC0838
V+ ZEN
VREF
DOUT
SARS
VCC-20
DGND-10
+5V
.1uf
CU301
+5V
SD00
/ADC0838
SDI
SCLOCK
SW301
+VBATT
J303
F301
1.5A
J304.1
12 VOLT
J304.2
C301
.1uf
C300
.1uf
U302
REF-05
VIN
VOUT
+5V REF TRIM
GND TEMP
+5VREF
R305
10K
CW
REF TEMP
VR303
IN OUT
LM7805T
CASE GND
C302
.1uf
C303
.1uf
+5V
100pf
C304
R302
1.2M
R303
100K
U303
LBI
LX
LBO
MAX643
VFB
VOUT
COMP
EXT
GND
C305
10uF
L301
Q301
VN01
SD302
1N5817
C306
100uF
L302
+18VUN
C307
100uF
C310
.1uF
VR301
IN OUT
LM78L15
GND
C311
.1uF
+15VL
C308
100uF
C309
100uF
C312
.1uF
VR302
GND
LM79L15
IN OUT
C313
.1uF
-15VL
SD301
1N5817
L303
-18VUN


FIG. 10A

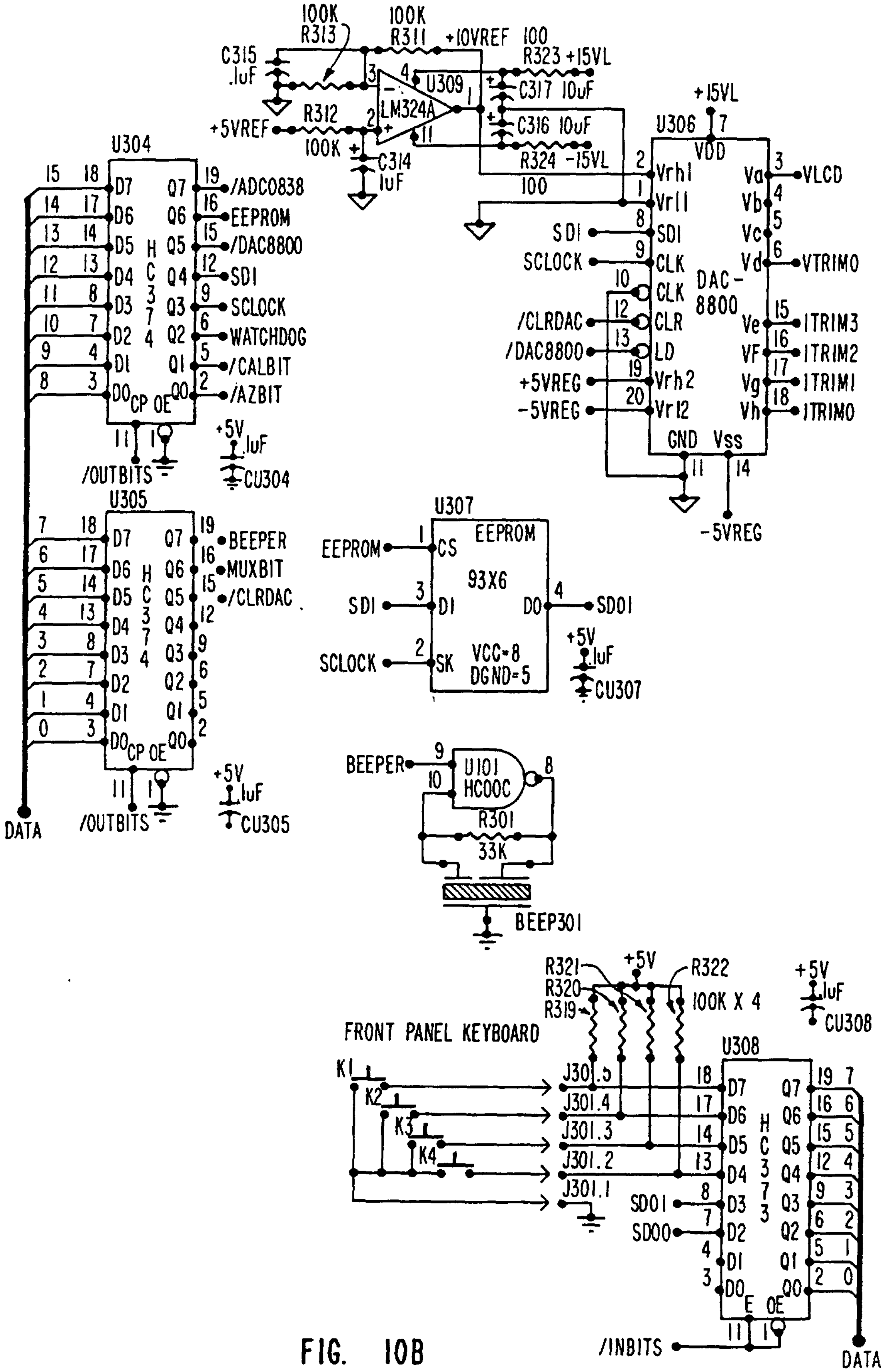

100K
R313
100K
R311
+10VREF
100
R323 +15VL
C315
.1uF
U309
LM324A
C317 10uF
C316 10uF
+5VREF
R312
100K
C314
1uF
R324 -15VL
100
+15VL
U306
VDD
Vrh1
Vrl1
SDI
CLK
CLK
CLR
LD
Vrh2
Vrl2
DAC-8800
Va
Vb
Vc
Vd
Ve
VF
Vg
Vh
VLCD
VTRIMO
ITRIM3
ITRIM2
ITRIM1
ITRIMO
GND Vss
SDI
SCLOCK
/CLRDAC
/DAC8800
+5VREG
-5VREG
-5VREG
U304
HC374
/ADC0838
EEPROM
/DAC8800
SDI
SCLOCK
WATCHDOG
/CALBIT
/AZBIT
/OUTBITS
+5V
.1uF
CU304
U305
HC374
BEEPER
MUXBIT
/CLRDAC
/OUTBITS
+5V
.1uF
CU305
DATA
U307
EEPROM
93X6
VCC=8
DGND=5
EEPROM
SDI
SCLOCK
SDO1
+5V
.1uF
CU307
BEEPER
U101
HC00C
R301
33K
BEEP301
R321
R320
R319
+5V
R322
100K X 4
+5V
.1uF
CU308
FRONT PANEL KEYBOARD
K1
K2
K3
K4
J301.5
J301.4
J301.3
J301.2
J301.1
SDO1
SDO0
U308
HC373
/INBITS
DATA


FIG. 10B

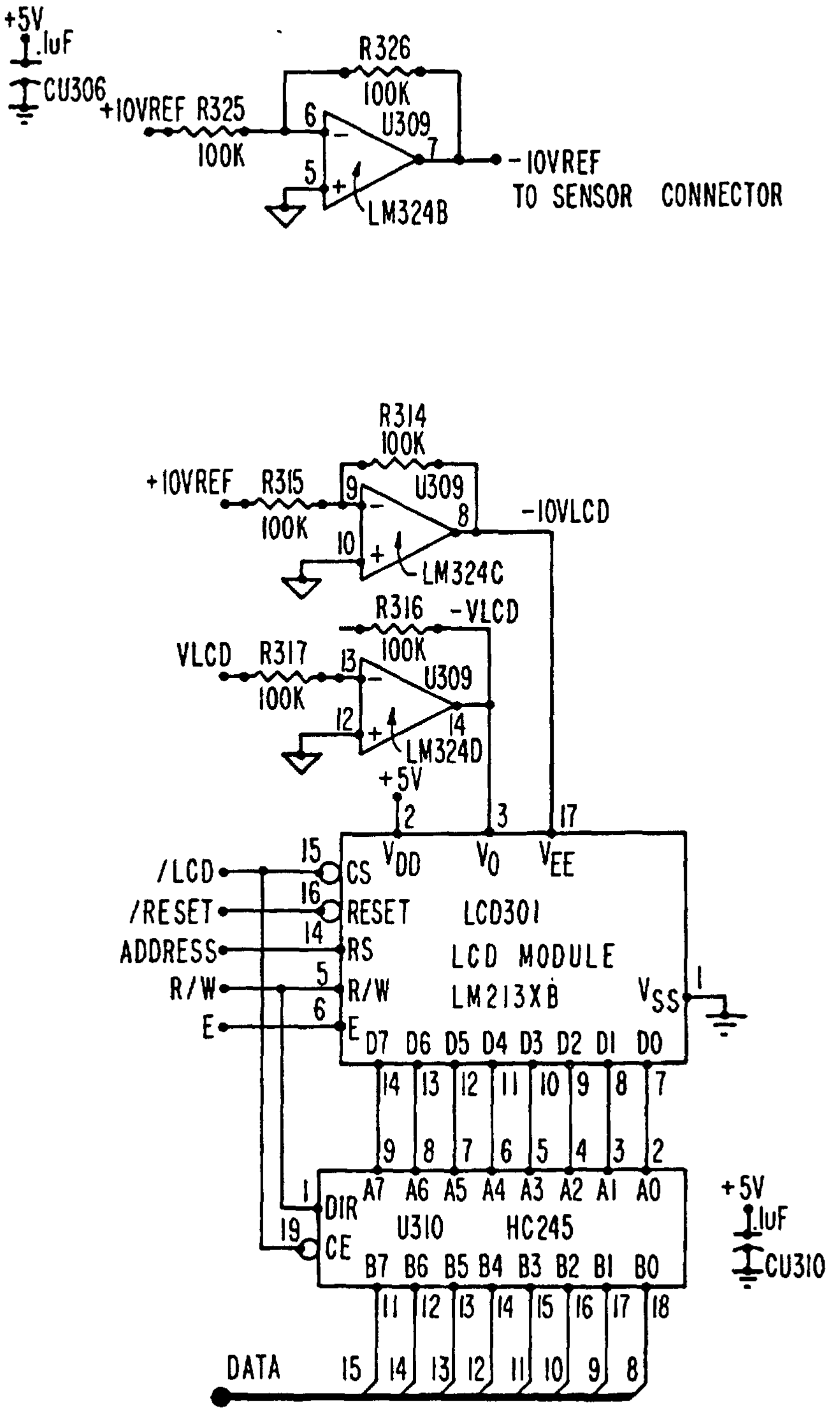

+5V
.luF
CU306
+10VREF
R325
100K
R326
100K
U309
6
5
7
LM324B
-10VREF
TO SENSOR CONNECTOR
R314
100K
+10VREF
R315
100K
9
10
8
LM324C
-10VLCD
R316
-VLCD
100K
VLCD
R317
100K
13
12
14
LM324D
+5V
2
3
17
15
16
14
5
6
/LCD
/RESET
ADDRESS
R/W
E
CS
RESET
RS
R/W
E
VDD
V0
VEE
LCD301
LCD MODULE
LM213XB
VSS
1
D7 D6 D5 D4 D3 D2 D1 D0
14 13 12 11 10 9 8 7
9 8 7 6 5 4 3 2
A7 A6 A5 A4 A3 A2 A1 A0
DIR
CE
U310
HC245
B7 B6 B5 B4 B3 B2 B1 B0
11 12 13 14 15 16 17 18
+5V
.luF
CU310
DATA
15 14 13 12 11 10 9 8


FIG. 10C

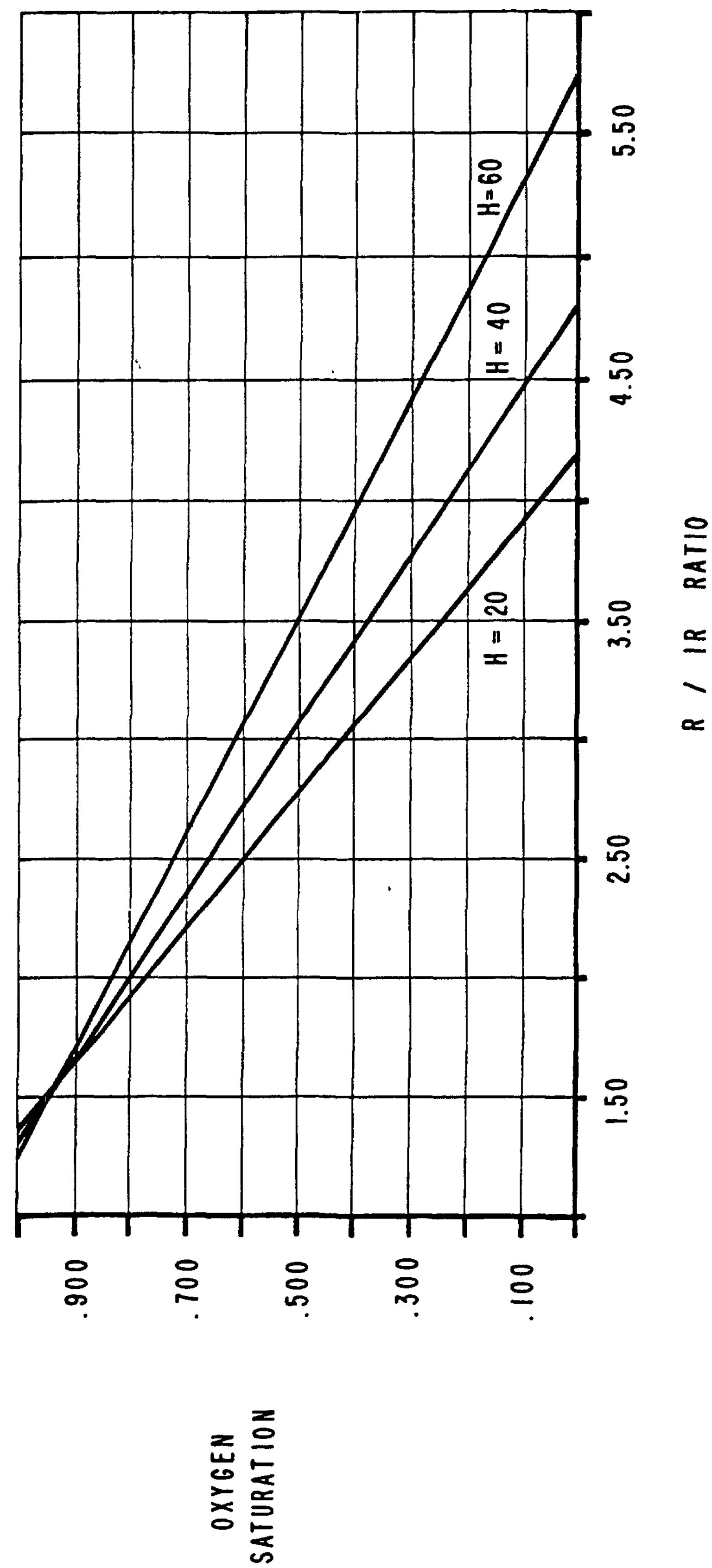
H=60
H = 40
H = 20
1.50
2.50
3.50
4.50
5.50
R / IR RATIO
.900
.700
.500
.300
.100
OXYGEN
SATURATION

FIG. 11

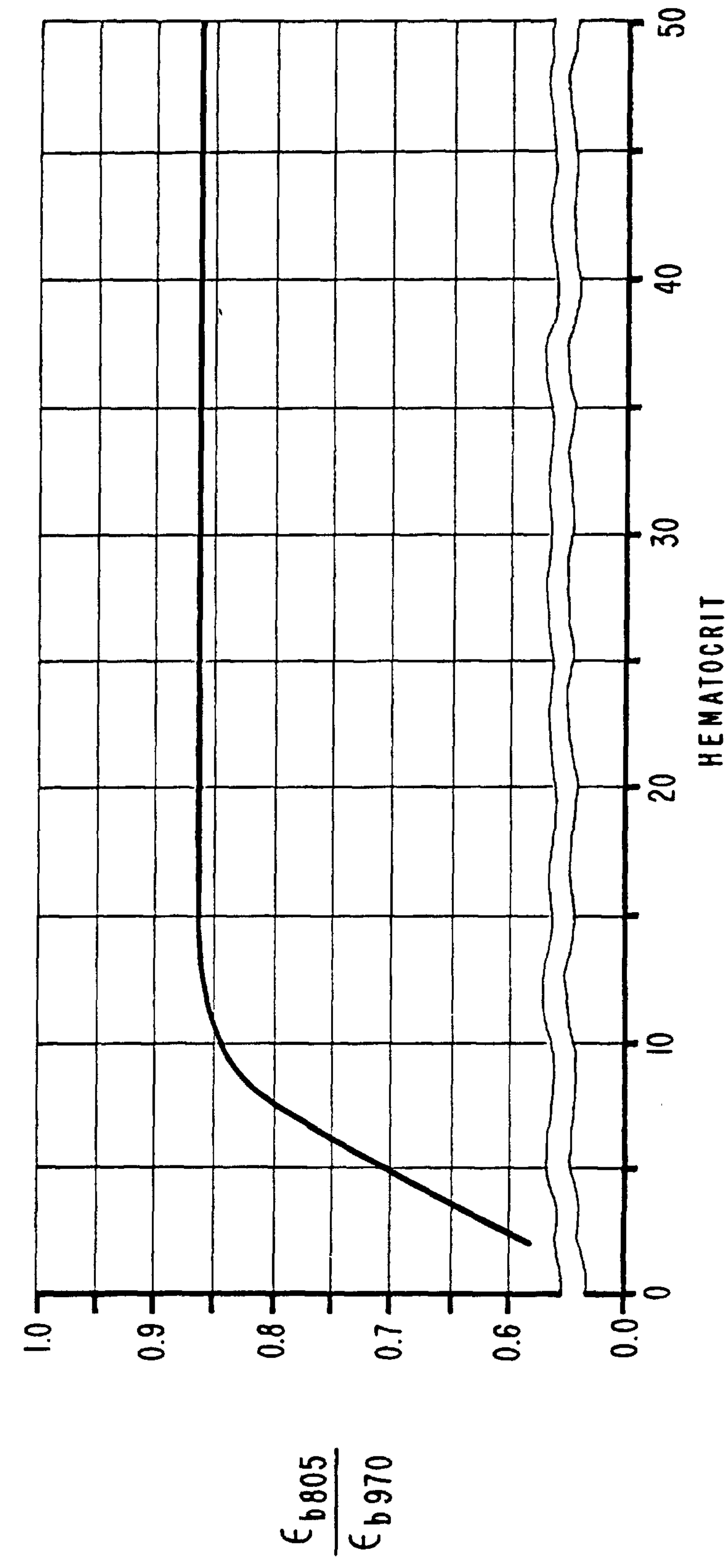
1.0
0.9
0.8
0.7
0.6
0.0
0
10
20
30
40
50
HEMATOCRIT
$\frac{\epsilon_{b805}}{\epsilon_{b970}}$

FIG. 12

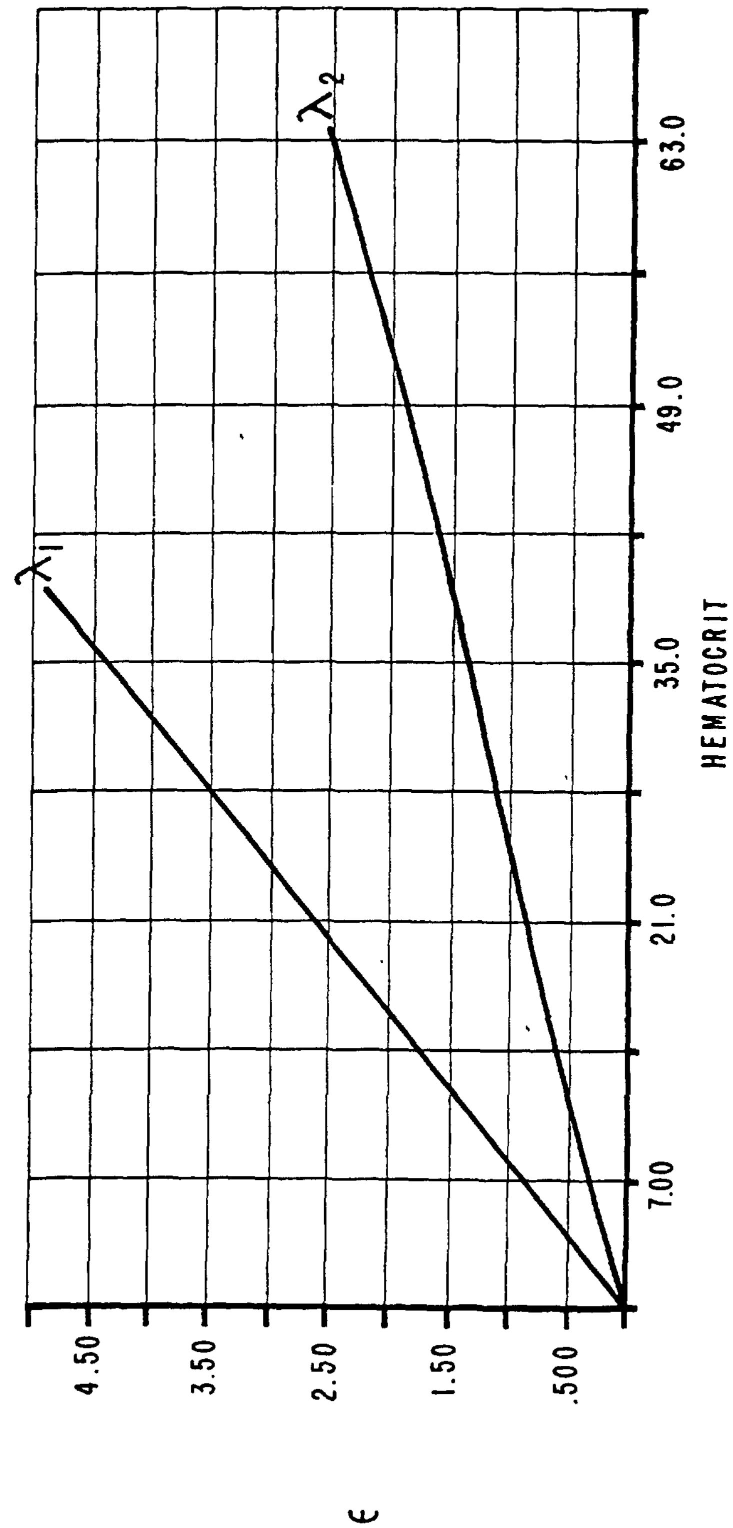


FIG. 13A

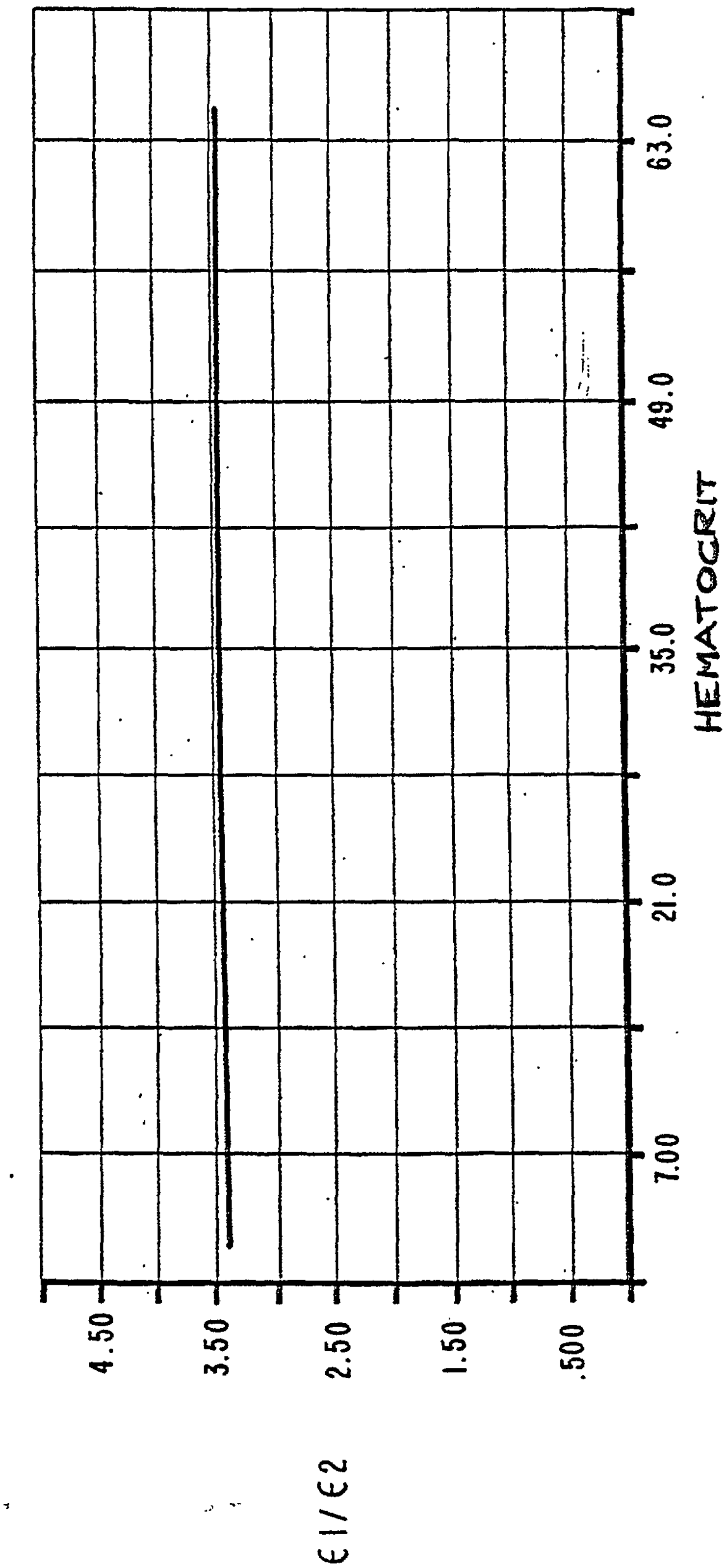


FIG. 13B

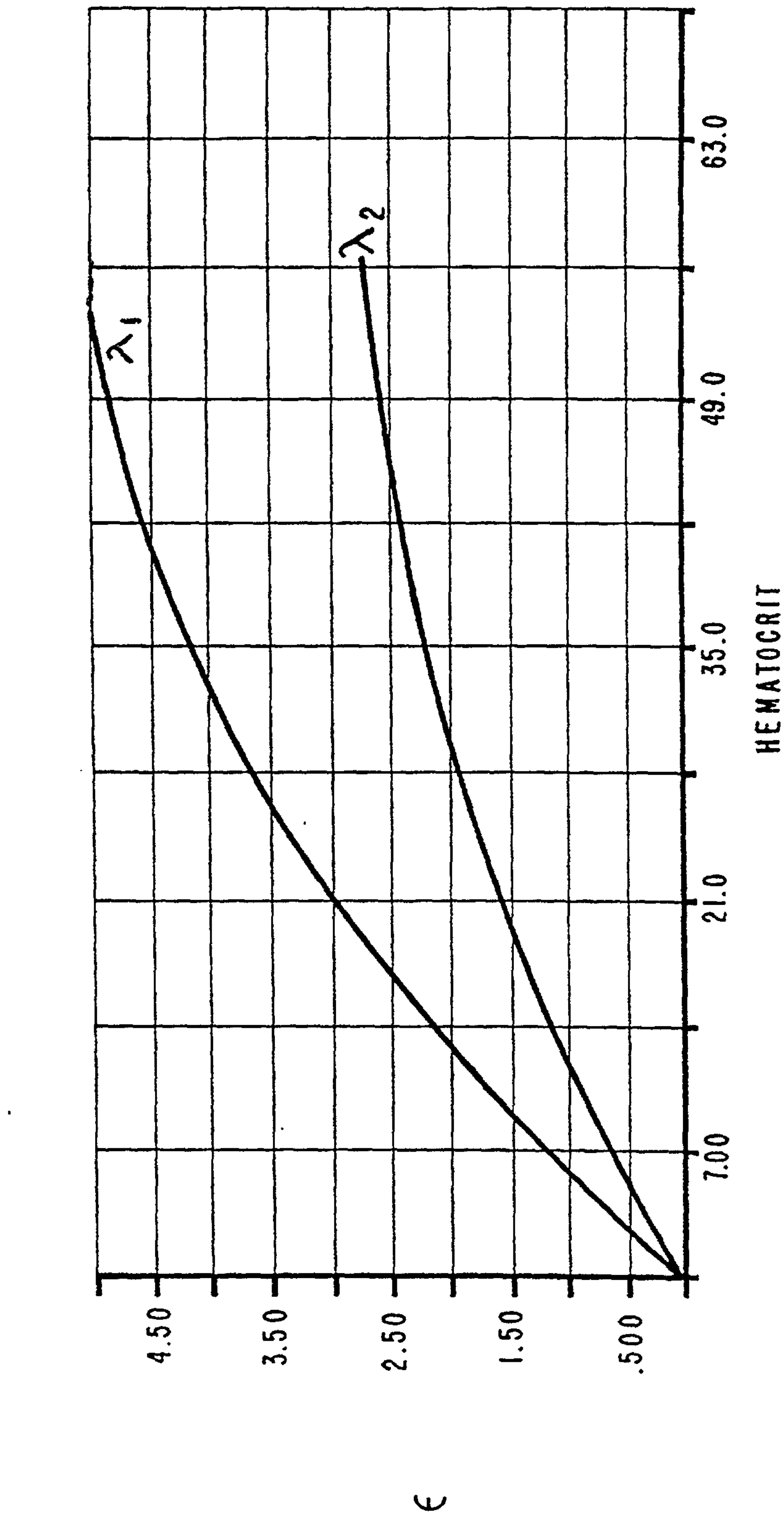


FIG. 14A

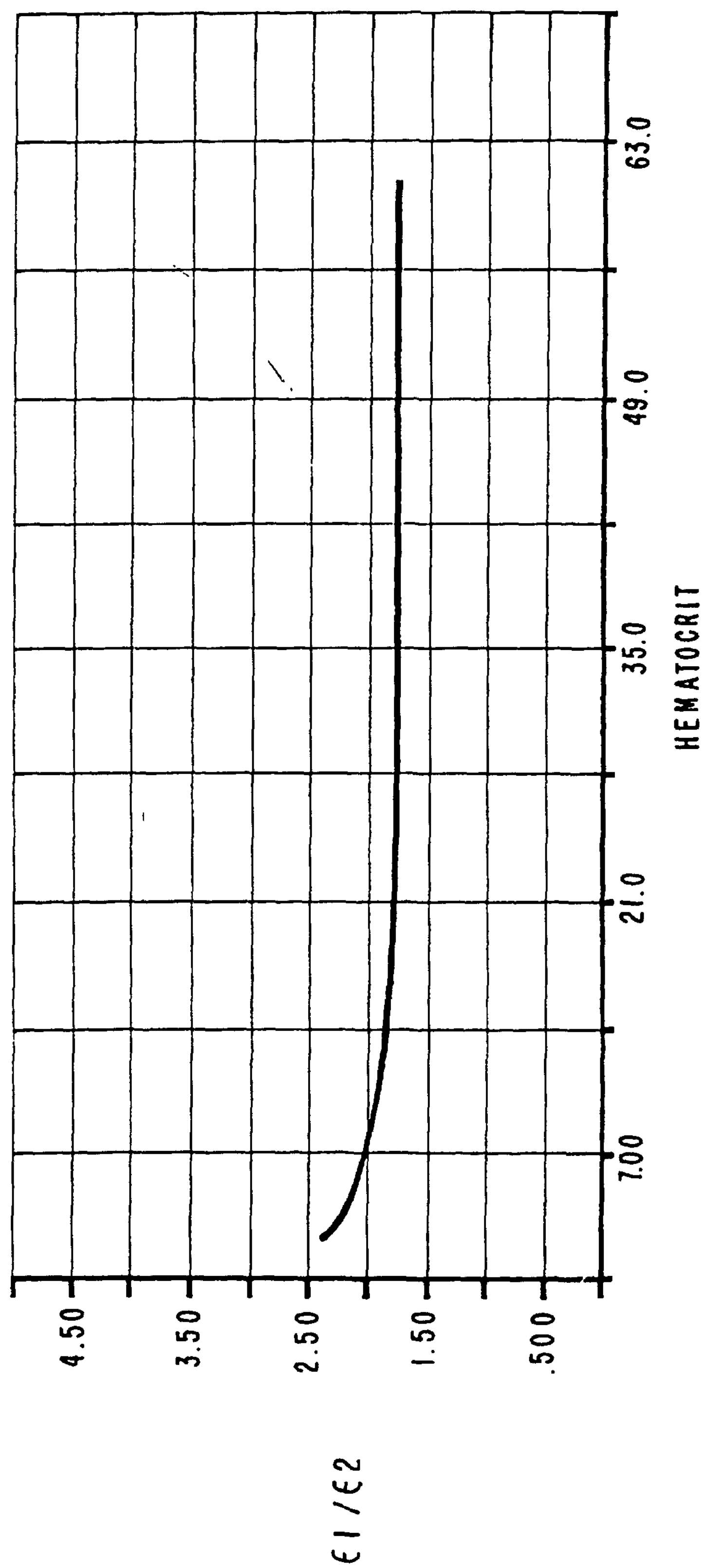


FIG. 14B

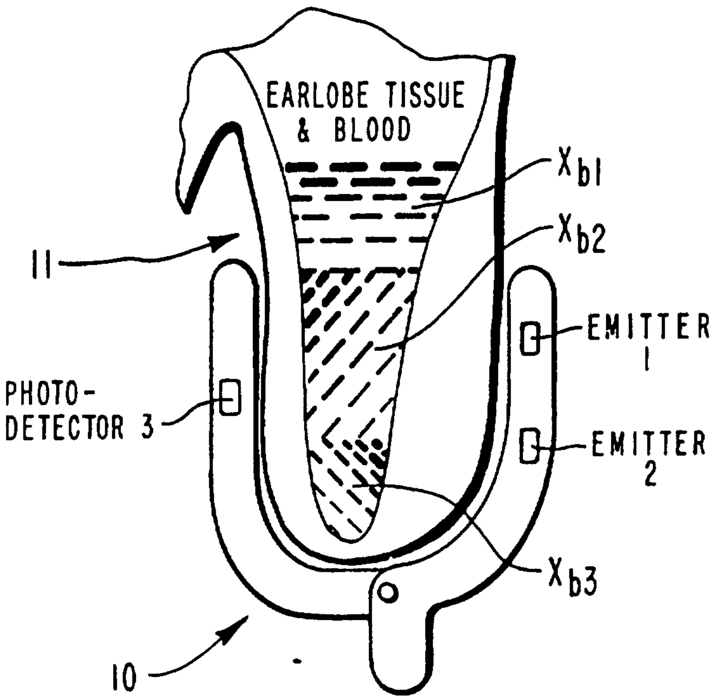
EARLOBE TISSUE
& BLOOD
Xb1
Xb2
11
EMITTER
1
PHOTO-
DETECTOR 3
EMITTER
2
Xb3
10

FIG. 15

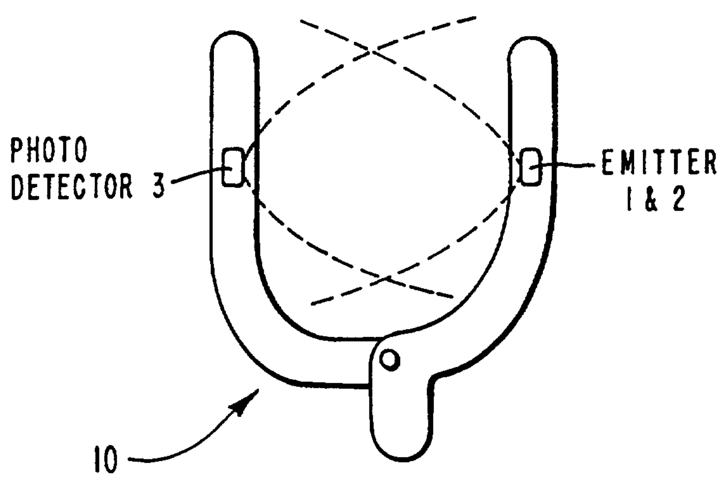
PHOTO
DETECTOR 3
EMITTER
1 & 2
10

FIG. 16